# FASCICULE DE BREVET EUROPEEN

(11) **EP 0 915 973 B1**
(45) Date de publication et mention de la délivrance du brevet: **01.07.2009**
(21) Numéro de dépôt: 97936721.6
(22) Date de dépôt: 31.07.1997
(51) Int. Cl.: C12N 15/12, C07K 14/705, C12Q 1/68, G01N 33/566, G01N 33/50, C07K 16/28, A61K 31/70, A61K 39/395

(54) **GENE IMPLIQUE DANS LE CADASIL, METHODE DE DIAGNOSTIC ET APPLICATION THERAPEUTIQUE**
IM CADASIL BEZOGENES GEN, DIAGNOSTIKUMS-VERFAHREN UND THERAPEUTISCHE VERWENDUNG
GENE INVOLVED IN CADASIL, METHOD OF DIAGNOSIS AND THERAPEUTIC APPLICATION

(30) Priorité: 01.08.1996 FR 9609733; 16.04.1997 FR 9704680
(43) Date de publication de la demande: 19.05.1999
(73) Titulaire: INSTITUT NATIONAL DE LA SANTE ET DE LA RECHERCHE MEDICALE (INSERM), 75013 Paris (FR); ASSISTANCE PUBLIQUE, HOPITAUX DE PARIS, 75004 Paris (FR)
(72) Inventeur: TOURNIER-LASSERVE, Elisabeth, F-75016 Paris (FR); JOUTEL, Anne, F-75005 Paris (FR); BOUSSER, Marie-Germaine, F-75014 Paris (FR); BACH, Jean-François, F-75015 Paris (FR)
(74) Mandataire: Warcoin, Jacques
(86) Numéro de dépôt international: PCT/FR1997/001433
(87) Numéro de publication internationale: WO 1998/005775

(56) Documents cités:
- CA-A- 2 116 628
- LARSSON C ET AL: "The human NOTCH1, 2, and 3 genes are located at chromosome positions 9q34, 1p13-p11, and 19p13.2-p13.1 in regions of neoplasia-associated translocation." GENOMICS, (1994 NOV 15) 24 (2) 253-8., XP000670019
- LARDELLI, M. ET AL.: "The novel Notch homologue mouse Notch3 lacks specific epidermal growth factor-repeats and is expressed in proliferating neuroepithelium" MECHANISMS OF DEVELOPMENT, vol. 46, mai 1994, pages 123-136, XP000670102
- LINDSELL, C. ET AL.: "Expression patterns of Jagged, Delta1, Notch1, Notch2, and Notch3 genes identify ligand-receptor pairs that may function in neural development." MOLECULAR AND CELLULAR NEUROSCIENCES, (1996) 8 (1) 14-27., XP000670030
- DUCROS A ET AL: "CEREBRAL AUTOSOMAL DOMINANT ARTERIOPATHY WITH SUBCORTICAL INFARCTS AND LEUKOENCEPHALOPATHY, GENETIC HOMOGENEITY, AND MAPPING OF THE LOCUS WITHIN A 2-CM INTERVAL" AMERICAN JOURNAL OF HUMAN GENETICS, vol. 58, no. 1, 1 janvier 1996, pages 171-181, XP000573967
- JOUTEL, A. ET AL.: "IDENTIFICATION OF EXPRESSED SEQUENCES FROM THE CADASIL REGION ON 19P" AMERICAN JOURNAL OF HUMAN GENETICS, (OCT 1995) VOL. 57, NO. 4, SUPP. S, PP. A342, ABR G 1985., XP000670015
- OPHOFF, R. ET AL.: "GENE FOR FAMILIAL HEMIPLEGIC MIGRAINE ON CHROMOSOME 19P13" AMERICAN JOURNAL OF HUMAN GENETICS, (OCT 1995) VOL. 57, NO. 4, SUPP. S, PP. A222, ABR G 1284., XP000670014
- JOUTEL A ET AL: "Notch3 mutations in CADASIL, a hereditary adult-onset condition causing stroke and dementia." NATURE, (1996 OCT 24) 383 (6602) 707-10., XP002029077
- JOUTEL, A. ET AL.: "Identification of the CADASIL gene." STROKE 28 (1). JANVIER 1997. 246, XP000670018 & 22ND INTERNATIONAL JOINT CONFERENCE ON STROKE AND CEREBRAL CIRCULATION, ANAHEIM, CALIFORNIA, USA, 6 AU 8 F VRIER 1997.,
- LARDELLI M ET AL: "EXPRESSION OF THE NOTCH 3 INTRACELLULAR DOMAIN IN MOUSE CENTRAL NERVOUS SYSTEM PROGENITOR CELLS IS LETHAL AND LEADS TO DISTURBED NEURAL TUBE DEVELOPMENT" MECHANISMS OF DEVELOPMENT, vol. 59, no. 2, 1996, pages 177-190, XP000670016

## Description

La présente invention concerne la mise en évidence de l'implication de la protéine Notch3 dans le CADASIL permettant ainsi la réalisation, notamment, d'un diagnostic d'une prédisposition à certains troubles neurologiques, en particulier le CADASIL, et de modèles permettant de tester les thérapies possibles pour ce type de pathologie.

Le CADASIL ou "Cerebral Autosomal Dominant Arteriopathy with Subcortical Infarts and Leukoencephalopathy" a récemment été identifié comme une cause d'attaques cérébrales et de démence dont les caractéristiques principales incluent des infarctus sous-corticaux récidivants, des migraines et une démence vasculaire, en association avec des images en IRM objectivant des anomalies diffuses de la substance blanche cérébrale.

L'examen anatomopathologique montre de multiples petits infarctus cérébraux profonds, une leucoencéphalopathie et une angiopathie non athéroscléreuse et non amyloïde impliquant essentiellement les petites artères cérébrales.

Comme son nom l'indique, CADASIL est une maladie héréditaire à caractère autosomique dominant. Pour plus d'informations on trouvera notamment une étude du spectre clinique de CADASIL dans H. Chabriat et al., The Lancet, vol. 346, 7 octobre 1995.

Cette maladie fortement incapacitante et très souvent léthale est probablement restée jusqu'ici largement non diagnostiquée en tant que telle ; l'étude d'une centaine de familles depuis 1993 montre que la plupart du temps des diagnostics erronés étaient donnés au patient (sclérose en plaques, maladie d'Alzheimer, etc.). Les études actuelles tendraient à démontrer qu'il s'agit d'une affection beaucoup plus répandue qu'on ne l'imaginait lors de sa mise en évidence.

Les recherches poursuivies actuellement ont pour objectifs la mise au point d'outils diagnostiques de la maladie, et, grâce notamment aux modèles et aux possibilités offertes par le génie génétique, la mise au point d'une thérapie éventuellement de substitution.

Le gène impliqué dans CADASIL a été localisé sur le chromosome 19 et une localisation plus fine est notamment mentionnée dans deux demandes de brevet ayant les mêmes inventeurs.

On a maintenant pu identifier le gène impliqué dans le CADASIL qui est le gène *Notch3*.

La mise en évidence de l'implication de *Notch3* dans CADASIL a pu être réalisée compte tenu des précédents encadrements qui avaient été mentionnés, notamment dans les demandes de brevet en cause, le premier intervalle (taille 14 cM) était D19S221-D19S215 (première demande de brevet), puis le deuxième intervalle (taille 2 cM) était D19S226-D19S199 (seconde demande de brevet). La région d'intérêt a été clonée dans un contig (séquence nucléotidique continue) de BAC et de YAC et sa taille a été estimée à 800 kb. L'analyse de cette région à l'aide d'enzymes de restriction a montré une très forte densité de sites NotI, EagI et SacII qui a suggéré la présence de nombreux gènes. Parmi les nombreux transcrits identifiés par sélection d'ADNc, un transcrit a montré une très forte homologie avec une séquence située à l'extrémité 5' codante du gène de souris *Notch*3. Comme d'autres éléments d'analyse ont semblé corroborer cette présence du gène *Notch3* dans cette situation, celui-ci a été considéré comme étant un bon gène candidat par sa position dans l'intervalle d'intérêt.

Les études comparatives effectuées sur les familles CADASIL connues en comparaison avec des sujets sains ont permis d'identifier des mutations sur ce gène *Notch3* chez un grand nombre de sujets CADASIL alors que de telles mutations n'étaient pas observées sur les sujets sains analysés. Comme, enfin, la coségrégation de ces mutations avec le phénotype malade a pu être démontrée au sein des familles atteintes, l'implication du gène *Notch3* dans le CADASIL devenait incontestable.

Toutes les mutations ponctuelles observées aboutissent à la création ou à la disparition d'une cystéine dans un des domaines EGF de cette protéine. Ces mutations sont clusterisées pour une grande partie d'entre elles dans les six premiers EGFs. La clusterisation des mutations revêt une importance certaine en termes de diagnostic notamment pour la recherche « séquentielle » de ces mutations.

Par ailleurs, toutes ces mutations aboutissent à la présence d'un nombre impair de cystéines dans un des EGF (soit sept, soit cinq cystéines) au lieu des six cystéines normalement présentes. Ces mutations pourraient ainsi résulter en la formation de ponts disulfures aberrants soit en intra soit en intermoléculaire (et dans ce cas à la formation d'homo ou d'hétérodimères).

Le rôle d'une dimérisation, normale ou anormale, dans le fonctionnement de récepteurs, en particulier leur activation, est bien connu.

Les gènes *Notch* sont connus depuis fort longtemps, notamment chez la drosophile et l'on connaît leur équivalent chez les vertébrés, en particulier chez la souris. Son nom anglais "notch", c'est-à-dire "encoche", provient du fait que certaines mutations de ce gène produisent une encoche dans les ailes des mouches. L'article de Spyros Artavanis-Tskanas et al., Science 268, 225 (1995) ainsi que les références qu'il contient indiquent que les protéines Notch sont essentiellement impliquées, notamment chez la drosophile, dans la spécification de la destinée cellulaire au cours du développement, et, bien que la protéine soit toujours exprimée dans les organismes adultes, ses fonctions chez ces derniers restent inconnues. Plus précisément, il apparaît que le produit du gène *Notch*3, ci-après "protéine Notch3", est un récepteur cellulaire qui commande une cascade d'événements cellulaires et dont la mutation conduit nécessairement à des dérèglements plus ou moins importants dans cette cascade, pouvant aboutir à de nombreux autres troubles neurologiques, notamment cérébro-vasculaires.

Il convient de rappeler que, si dans ce qui va suivre on s'intéresse plus particulièrement aux troubles neurologiques, notamment les troubles de type cérébrovasculaires et tout particulièrement le CADASIL, il est probable, compte tenu de la fonction de récepteur cellulaire du produit du gène *Notch3*, qu'une altération de ce récepteur puisse conduire à une désorganisation de son interaction avec divers ligands mais également avec les différents partenaires impliqués dans la cascade de transduction. Il faut tenir compte, en outre, du fait que la protéine Notch3 pourrait avoir d'autres fonctions qui n'ont, pour l'instant, pas été mises en évidence. Dans ces conditions, il est très probable que des affections présentant des analogies avec le CADASIL puissent également être impliquées en cas de mutation dans le gène *Notch3*.

Parmi les maladies concernées, il faut citer les formes sporadiques de CADASIL, c'est-à-dire intervenant sans antécédents familiaux mais à la suite d'une néomutation. *Notch3* pourrait par ailleurs être impliqué dans d'autres affections classables en différents groupes :

### Migraine hémiplégique et migraine

On a montré qu'au moins un des gènes impliqués dans la migraine hémiplégique familiale (MHF), forme autosomique dominante de migraine avec aura, était localisé dans la même région du chromosome 19 que le gène de CADASIL. Il est à noter que plus de 30% des patients atteints de CADASIL, affection caractérisée par la survenue répétée d'accidents vasculaires cérébraux et d'une démence vasculaire, souffraient de migraine avec aura. Or cette dernière n'est observée que dans 5% environ de la population ; c'est cette observation qui a conduit à tester l'implication du gène de CADASIL dans les mécanismes de cette affection. L'implication de ce gène dans une forme de migraine avec ou sans aura présenterait un intérêt diagnostique et thérapeutique considérable en raison de la fréquence de la migraine avec aura et de la migraine sans aura dans la population générale.

### Autres pathologies vasculaires (infarctus cérébraux) et/ou démentielles d'étiologie inconnue

Ce groupe correspond à un nombre très important de patients dans les services de neurologie, de psychiatrie et de médecine interne et il est tout à fait raisonnable de penser que *Notch3* ou un partenaire de cette voie de signalisation puisse être impliqué dans ces affections pour les raisons évoquées précédemment.

### Ataxie paroxytique familiale

La situation est la même que pour la MHF. On a localisé un gène responsable de cette affection dans la même région du chromosome 19 et *Notch3* pourrait être en cause dans cette affection.

Par ailleurs, les mutations de ce gène sont responsables d'anomalies développementales très connues dans d'autres espèces ainsi que de pathologies de type néoplasique. Des syndromes malformatifs et/ou néoplasiques dans lesquels serait mis en évidence un remaniement de la région qui contient ce gène seraient des candidats majeurs pour la recherche d'une implication de ce gène dans leur physiopathologie.

Ces troubles pourront être rassemblés sous le nom de "troubles liés au récepteur Notch3".

Dans certains cas, il pourra s'agir de troubles ayant une origine multigénique mais dans lesquels les modifications de Notch3 contribueraient à la survenue de la pathologie ou à son aggravation.

La présente invention décrit une séquence nucléotidique isolée, **caractérisée en ce qu**'elle est choisie parmi :
a) les séquences codant pour la protéine Notch3 humaine et ses variants alléliques,
b) les séquences codant pour un fragment de ces protéines et ayant au moins 10 bases,
c) les séquences génomiques Notch3 humain et ses allèles,
d) les séquences présentant au moins 80%, et de préférence au moins 90%, d'homologie avec les séquences (a) et (c),
e) les fragments des séquences (c) ou (d) ayant au moins 10 bases,
f) les séquences qui s'hybrident avec une séquence de (a) à (e).

Il doit être compris que la présente invention ne concerne pas les séquences nucléotidiques génomiques dans leur environnement chromosomique naturel, c'est-à-dire à l'état naturel, il s'agit de séquences qui ont été isolées, c'est-à-dire qu'elles ont été prélevées directement ou indirectement, par exemple par copie (ADNc), leur environnement ayant été au moins partiellement modifié.

Ainsi, il peut s'agir aussi bien d'ADNc que d'ADN génomique partiellement modifié ou porté par des séquences au moins partiellement différentes des séquences les portant naturellement.

Ces séquences pourront également être qualifiées de "non naturelles".

Par "séquence nucléique", on entend un fragment d'ADN et/ou d'ARN naturel isolé, ou de synthèse, désignant un enchaînement précis de nucléotides, modifiés ou non, permettant de définir un fragment, un segment ou une région d'un acide nucléique.

Par "variant allélique" de la protéine, on entend désigner l'ensemble des protéines mutées et des polymorphismes pouvant exister chez l'être humain, obtenus notamment par troncature, substitution, délétion ou addition de résidus d'amino-acides, ainsi que les variants artificiels.

Les fragments de séquences nucléiques peuvent notamment coder pour des domaines de la protéine ou bien être utilisés comme sonde ou comme amorce dans des procédés de détection ou d'identification ou d'amplification. Ces fragments présentent une taille minimale de 10 bases et on préférera des fragments de 20 bases, et de préférence 30 bases.

L'homologie est uniquement de type statistique, elle signifie que les séquences présentent au minimum 80%, et préférentiellement 90%, de nucléotides en commun.

Les conditions d'hybridation doivent permettre, selon l'invention, d'assurer au moins 95% d'homologie.

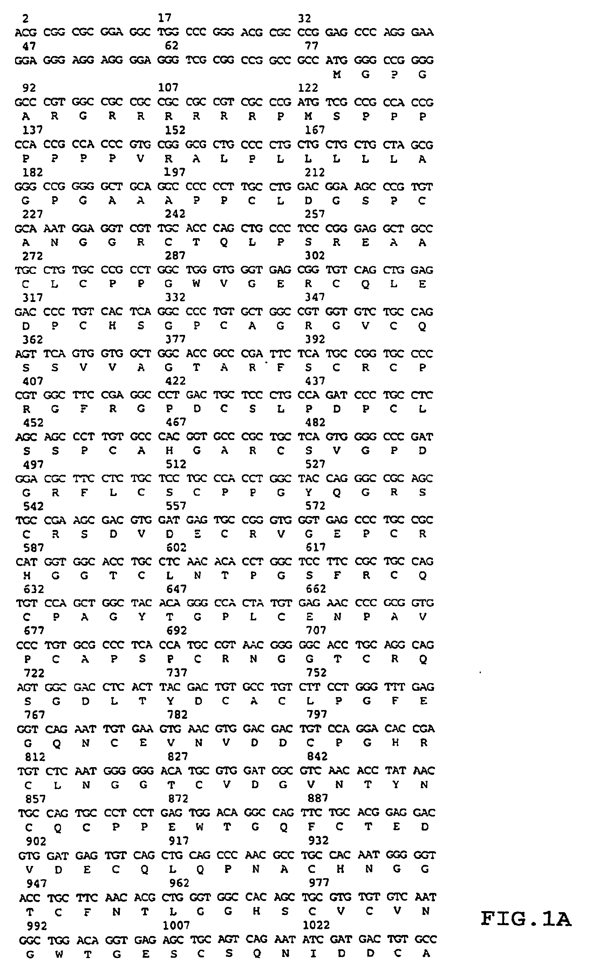

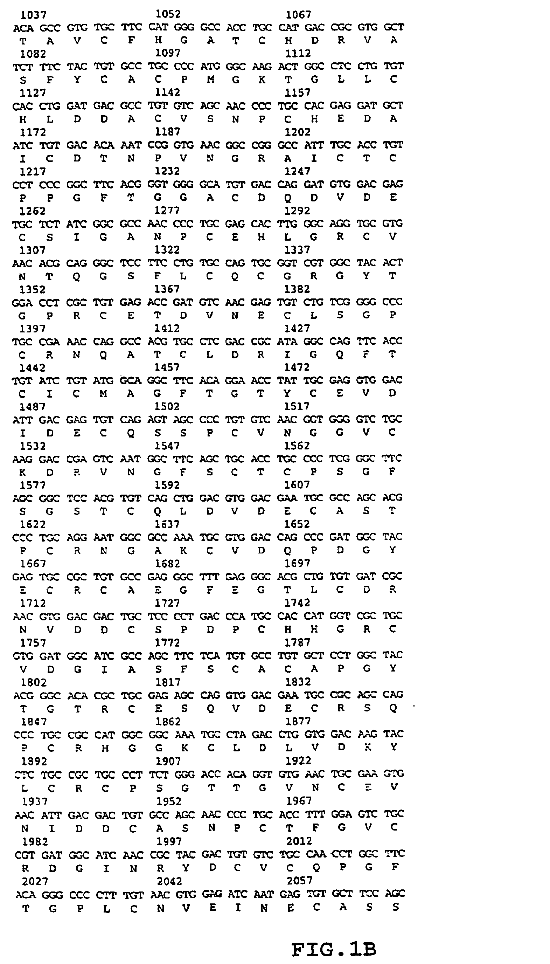

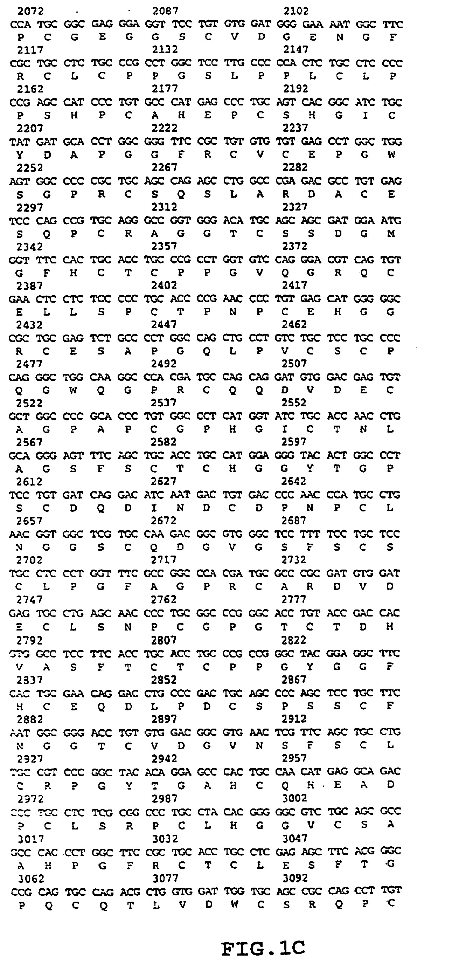

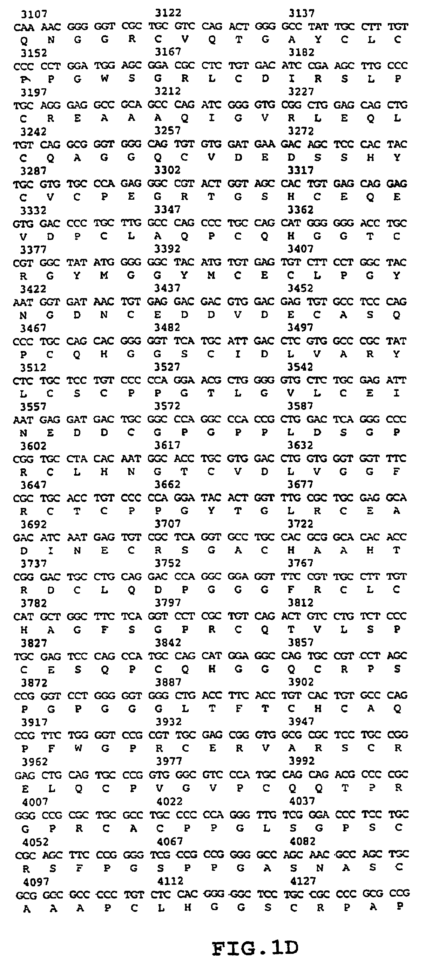

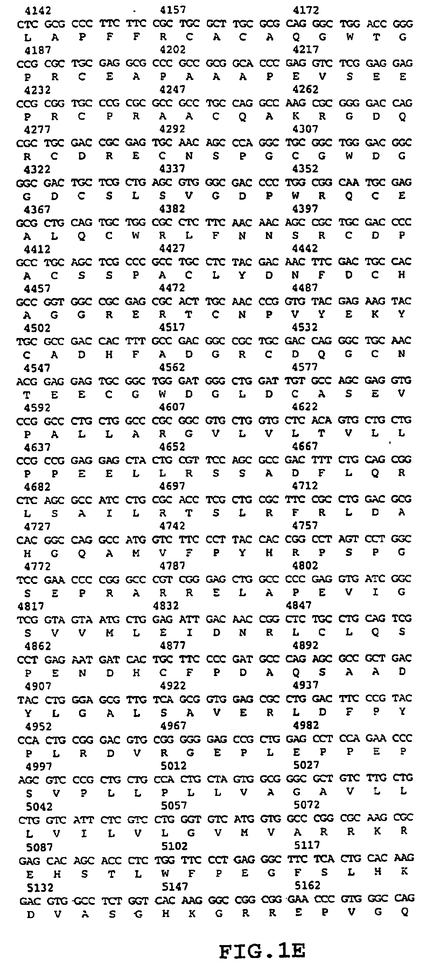

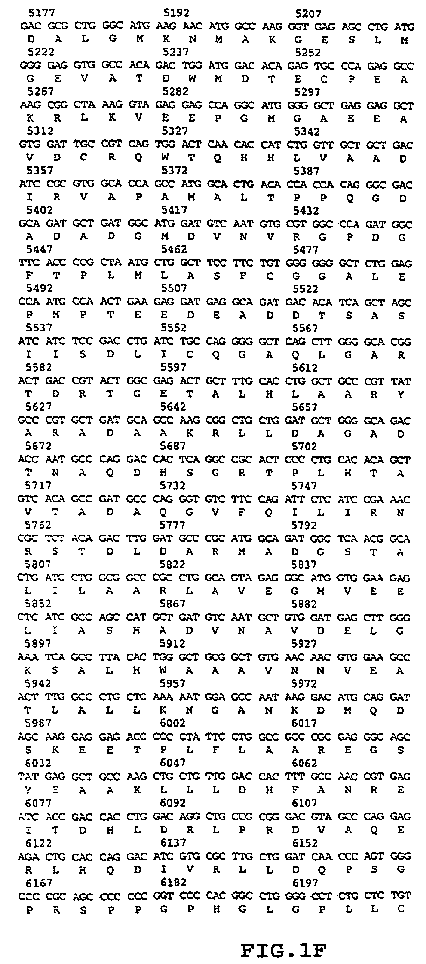

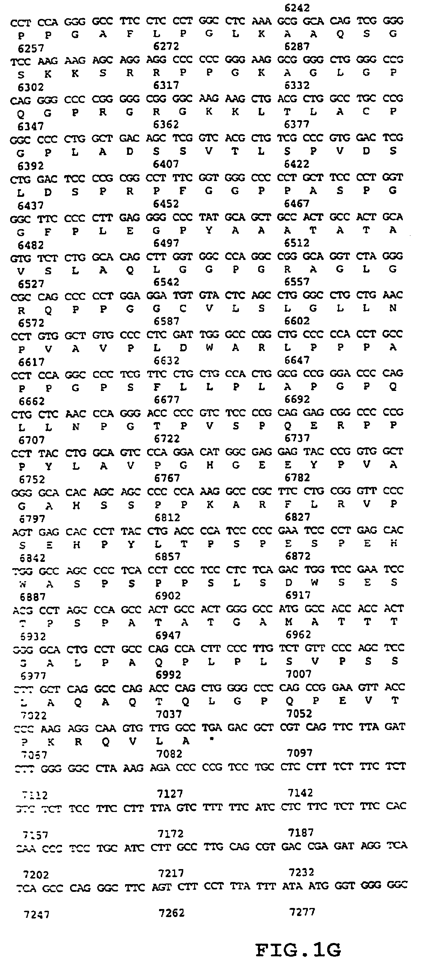

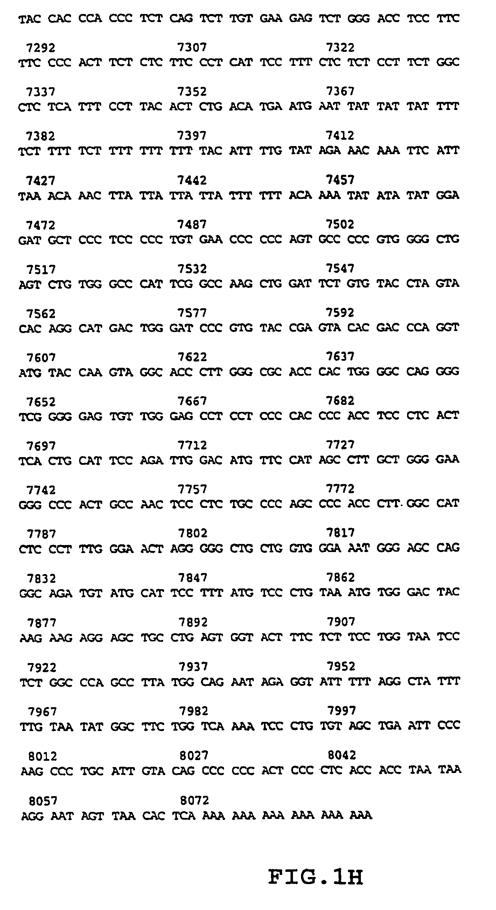

La figure 1 représente les séquences de Notch3 telles qu'elles ont été séquencées sur un génome normal.

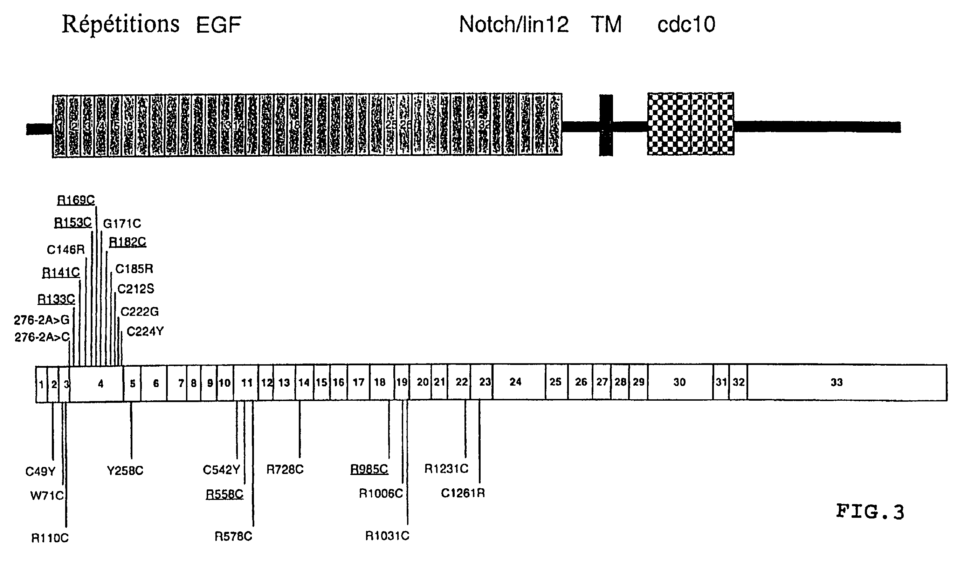

Les séquences sont identifiées par des références qui permettent de les situer les unes par rapport aux autres grâce à la figure 3.

Pour ce qui concerne les remarques particulières sur (a), (b), (c) et (d), les remarques précédentes s'appliquent.

L'invention décrit également des fragments de ces séquences, en particulier des séquences codant pour des polypeptides ayant gardé tout ou partie de l'activité de la protéine Notch3.

Parmi les séquences particulièrement intéressantes, il faut citer celles codant pour des domaines ou des combinaisons de domaines de la protéine Notch3, soit les séquences :
- répétitions "EGF"
- répétitions "Notch/lin 12"
- répétitions "cdc10/SW16"
ou la séquence transmembranaire.

Parmi les séquences intéressantes, figure notamment la séquence codée par le deuxième transcrit que l'on décrira dans ce qui va suivre, ledit transcrit ayant une taille estimée entre 1,3 à 2,4 kb.

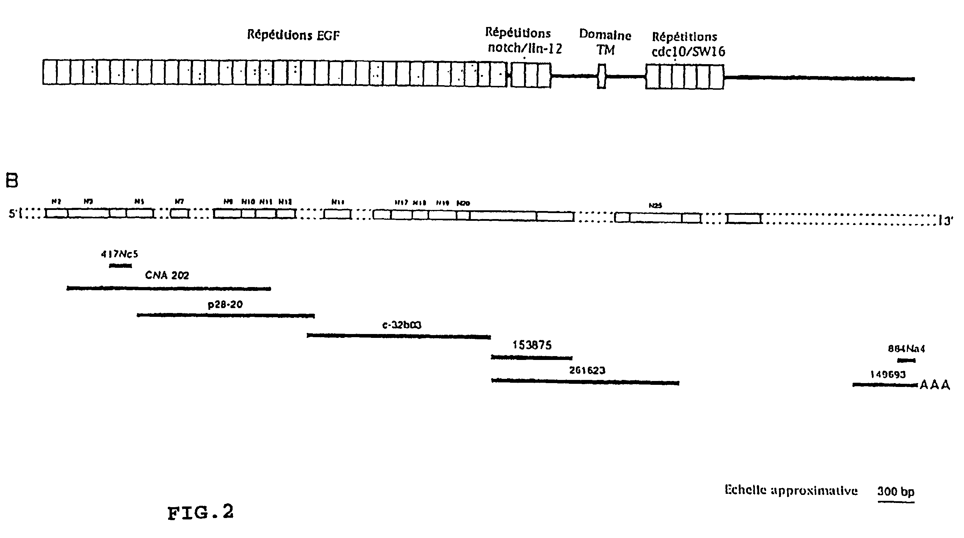

Ces séquences peuvent être identifiées en se reportant notamment à la figure 2 qui schématise l'organisation de Notch3.

Ces séquences partielles peuvent être utilisées pour de nombreuses applications, comme cela sera décrit ci-après, notamment pour effectuer des constructions protéiques de type Notch ou de types différents, mais également pour réaliser par exemple des protéines Notch-like tronquée qui serviront de leurre au ligand de Notch3 ou d'agoniste de la protéine.

On peut également prévoir d'utiliser ces séquences protéiques pour leurs effets propres, ainsi les domaines EGF sont présents dans d'autres protéines, notamment d'autres récepteurs, on pourra par exemple se reporter à Iain D. Campbell, Current Biology, 3: 385-392 (1993) pour d'autres d'applications des séquences EGF en cause.

La présente invention concerne une séquence nucléotidique choisie parmi :
a) les séquences codant pour un polypeptide comprenant les amino-acides selon la séquence de la figure 1,
b) la séquence nucléique de l'ADNc représenté à la figure 1 codant pour le polypeptide Notch3,
**caractérisée en ce que** les séquences telles que définies en a) ou en b) comportent au moins une mutation qui supprime une cystéine ou, au contraire, qui en fait apparaître sur ledit polypeptide Notch3 dans un de ces domaines EGF.

La présente invention décrit également les mutations pouvant intervenir dans les séquences promotrices et/ou régulatrices du gène *Notch3* humain, lesquelles peuvent avoir des effets sur l'expression de la protéine.

Des exemples de telles mutations seront décrits dans ce qui va suivre.

De façon générale, la présente invention s'intéresse aussi bien à la protéine Notch3 normale qu'aux protéines Notch3 mutées, ainsi qu'à leurs fragments et aux séquences d'ADN et d'ARN correspondantes, c'est-à-dire les allèles.

Il est à noter que l'étude en Northern blot de l'expression de ce gène dans des tissus humains met en évidence deux transcrits. L'un d'une taille estimée à 7,5 - 9,5 kb est présent dans tous les tissus testés ; l'autre, dont la taille est comprise entre 1,3 et 2,4 kb, n'est détecté que dans certaines parties du système nerveux central. La présente invention concerne ces deux transcrits.

Parmi les fragments nucléotidiques, il faut citer les séquences génomiques introniques du gène *Notch3* et plus particulièrement les séquences jonctions entre les introns et les exons, notamment tels qu'ils sont représentés dans le tableau A, et, enfin, la présente invention concerne l'ensemble des amorces qui peuvent être déduites des séquences nucléotidiques précédentes et qui peuvent permettre de les mettre en évidence en utilisant une méthode d'amplification telle que la méthode PCR, notamment celles figurant dans le tableau B.

La présente invention décrit également les séquences nucléotidiques qui peuvent comporter des nucléotides non naturels, notamment des nucléotides soufrés par exemple ou de structure α ou β.

Enfin, la présente invention décrit aussi bien les séquences ADN qu'ARN, de même que les ADN double brin correspondants.

Comme cela sera décrit ci-après, pour certaines applications, il peut être nécessaire de prévoir des constructions mixtes, protéine/ADN/composé chimique, notamment l'utilisation d'agents intercalants par exemple, il doit être compris que de tels composés sont couverts par le brevet comme comportant une séquence selon l'invention.

La présente invention décrit également les protéines polypeptides ou peptides correspondant aux séquences mentionnées précédemment, sous forme non naturelle, c'est-à-dire qu'elles ne sont pas prises dans leur environnement naturel mais obtenues par purification à partir de sources naturelles ou bien obtenues par recombinaison génétique, comme cela sera décrit ci-après.

L'invention décrit également les mêmes polypeptides ou protéines obtenus par synthèse chimique et pouvant comporter des amino-acides non-naturels.

La présente invention décrit les protéines recombinantes ainsi obtenues aussi bien sous forme glycosylée que non glycosylée et pouvant présenter ou non la structure tertiaire naturelle.

En particulier, la présente invention décrit les fragments de Notch3 qui présentent une activité similaire au récepteur total, notamment la ou les parties solubles dudit récepteur correspondant en particulier au domaine extracellulaire. Ceux-ci pourront être utilisés notamment comme leurre dans une thérapie, comme cela sera décrit ci-après.

La présente invention concerne également des vecteurs de clonage et d'expression comportant une séquence nucléotidique selon l'invention.

Ces vecteurs de clonage et d'expression pourront comporter des éléments assurant l'expression de la séquence dans une cellule hôte, notamment des séquences promotrices et des séquences de régulation efficaces dans ladite cellule (voir référence infra).

Le vecteur en cause pouvant être à réplication autonome ou bien destiné à assurer l'intégration de la séquence au sein des chromosomes de la cellule hôte.

Dans le cas de systèmes à réplication autonome, en fonction de la cellule hôte, procaryote ou eucaryote, on utilisera de préférence des systèmes de type plasmidique ou des systèmes viraux, les virus vecteurs pouvant être notamment des adénovirus, des poxvirus ou des virus herpétiques. L'homme de métier connaît les technologies utilisables pour chacun de ces virus (voir référence infra).

Lorsque l'on souhaitera l'intégration de la séquence dans les chromosomes de la cellule hôte, il sera nécessaire de prévoir de part et d'autre de la séquence nucléotidique à intégrer une ou plusieurs séquences provenant de la cellule hôte afin d'assurer la recombinaison. Il s'agit là également de procédés qui sont largement décrits dans la technique antérieure. On pourra, par exemple, utiliser des systèmes de type plasmidique ou viral ; de tels virus seront, par exemple, les rétrovirus ou les AAV (Associations Adénovirus Virus).

L'invention concerne également les cellules procaryotes ou eucaryotes transformées par un vecteur selon l'invention et ceci afin d'assurer l'expression d'une protéine Notch3.

Comme cela a été indiqué précédemment, la présente invention concerne également les protéines, peptides ou polypeptides obtenues par culture des cellules de l'invention ainsi transformées et récupération de la protéine exprimée, ladite récupération pouvant être effectuée de façon intracellulaire ou bien de façon extracellulaire dans le milieu de culture lorsque le vecteur a été conçu pour assurer l'excrétion de la protéine par le biais, par exemple, d'une séquence "leader", la protéine étant sous forme d'une pré-protéine ou prépro-protéine. Les constructions permettant la sécrétion des protéines sont connues, aussi bien pour des systèmes procaryotes que des systèmes eucaryotes.

Parmi les cellules utilisables pour la production de ces protéines, il faut citer bien entendu les cellules bactériennes, mais également les cellules de levure, de même que les cellules animales, en particulier les cultures de cellules de mammifère mais également les cellules d'insectes dans lesquelles on peut utiliser des procédés mettant en oeuvre des baculovirus par exemple (voir référence infra).

Les cellules ainsi obtenues peuvent permettre de préparer des protéines naturelles ou mutées Notch3, mais également des fragments de ces protéines, notamment des polypeptides pouvant correspondre aux différents domaines en cause.

Mais, les cellules transformées telles que décrites précédemment pourront également être utilisées à titre de modèle afin d'étudier les interactions entre le gène Notch et ses différents ligands ainsi que son influence sur les produits en aval du récepteur, mais surtout ils pourront être utilisés dans une application pour la sélection de produits interagissant avec le récepteur Notch3, naturel ou muté, à titre d'agoniste ou d'antagoniste de ce récepteur.

Ce type de modèle cellulaire peut être réalisé en mettant en oeuvre des techniques de génie génétique. Il s'agit, suivant le type de cellules que l'on désire utiliser, de cloner le gène en question sous sa forme normale ou sous sa forme mutée dans un vecteur d'expression, qu'il s'agisse d'un vecteur à réplication autonome ou d'un vecteur d'intégration, ledit vecteur comportant l'ensemble des éléments permettant l'expression du gène dans la cellule en cause, ou celle-ci ayant l'ensemble des éléments permettant l'expression de la séquence en cause.

On obtient ainsi des cellules eucaryotes ou procaryotes exprimant la ou les protéines Notch3 qui, compte tenu de ses caractéristiques, se situera comme une protéine transmembranaire dont la structure fine sera décrite dans ce qui va suivre, lesdites cellules pouvant alors constituer des modèles permettant de tester tout à la fois les interactions de différents ligands avec le produit de la protéine Notch3 ou de tester des produits chimiques de synthèse pouvant interagir avec le produit du gène *Notch3* et ce en les ajoutant dans le milieu de culture desdites cellules.

Il faut, en particulier, remarquer que les produits en question pourront aussi bien être des agents à activité antagoniste qu'agoniste.

L'utilisation de modèles cellulaires en vue de tester des composés pharmaceutiques est bien connue, là encore il n'y a pas lieu de détailler ce type de modèle.

Une autre application potentielle de la caractérisation de ce gène est la possibilité d'identifier des ligands potentiels de cette protéine, soit parce qu'ils ont une séquence conservée avec Notch3 humain, soit parce qu'ils interagissent avec Notch3 (méthodes d'affinité) ou des partenaires de cette voie de signalisation.

Ces modèles peuvent être de type in vitro, par exemple des cultures de cellules humaines, soit en culture normale, soit éventuellement sous forme d'organe isolé, comme par exemple certains types de vaisseaux que l'on pourra transformer afin de leur faire exprimer les phénotypes recherchés.

La présente invention décrit également des organismes tels que les animaux, en particulier des souris, exprimant le phénotype correspondant à Notch3 normal ou muté d'origine humaine. Là encore, ces animaux pourront être utilisés comme animaux modèles pour tester l'efficacité de certains produits pharmaceutiques.

La présente invention concerne également les produits obtenus par la mise en oeuvre des modèles cellulaires de l'invention.

Il est possible d'obtenir, en fonction du type d'interaction déterminée, des compositions thérapeutiques **caractérisées en ce qu**'elles comportent à titre de principe actif un composé à activité pro-Notch3, il pourra s'agir notamment de tout ou partie d'un polypeptide tels qu'ils ont été décrits précédemment ou bien d'un vecteur d'expression de ces mêmes polypeptides, ou bien encore de composés chimiques ou biologiques ayant une activité pro-Notch3, une activité Notch3-like ou induisant la production de Notch3 naturel.

Il sera possible également de mettre en évidence des compositions thérapeutiques dans lesquelles le principe actif aura une action anti-Notch3.

Il pourra s'agir, là encore, de protéines modifiées décrites précédemment qui pourront jouer le rôle de leurre, ou bien d'anticorps anti-Notch3, en particulier lorsque ces anticorps reconnaîtront les récepteurs mutés et pourront dans ces conditions bloquer l'activité du récepteur normal.

Il pourra s'agir également de produits chimiques ayant une activité anti-Notch3, soit des antagonistes de Notch3.

Dans certains cas, l'utilisation de certains des domaines de Notch3 peut permettre une approche thérapeutique bloquant l'activité du récepteur Notch3 lorsque celui-ci est muté en utilisant des récepteurs solubles qui serviront de leurres aux ligands naturels, dans d'autres cas, il sera possible, en exprimant la totalité du récepteur, d'assurer une thérapie de remplacement en utilisant, soit directement les protéines ou leurs fragments, ou bien en exprimant directement la protéine, notamment par le biais de la thérapie génique et en utilisant les vecteurs qui ont été décrits précédemment.

Dans le cadre de la thérapie génique, il est possible également de prévoir l'utilisation des séquences des gènes ou des ADNc précédemment décrits "nus", cette technique a notamment été développée par la société Vical, elle a montré qu'il était, dans ces conditions, possible d'exprimer la protéine dans certains tissus sans avoir recours au support d'un vecteur viral notamment.

Toujours dans le cadre de la thérapie génique, il est également possible de prévoir l'utilisation de cellules transformées ex-vivo, lesquelles pourront être ensuite réimplantées, soit telles quelles, soit au sein de systèmes de type organoïde, tel que cela est également connu dans l'état de la technique. On peut également envisager l'utilisation d'agent facilitant le ciblage de type cellulaire déterminé, la pénétration dans les cellules ou le transport vers le noyau.

Parmi les nombreux composés pharmaceutiques utilisables, il faut citer plus particulièrement, outre les ligands du produit de Notch3, les séquences sens ou anti-sens interagissant avec le gène *Notch3* normal ou muté, ou bien interagissant sur la régulation ou l'expression de ces gènes, lesdits produits pouvant également interagir en aval des produits d'expression induits par les récepteurs Notch3. Il faut de plus citer les séquences solubles correspondant à *Notch 3*.

Il faut également mentionner les anticorps monoclonaux bloquant les récepteurs Notch3, en particulier les récepteurs Notch3 mutés, et/ou bloquant les ligands correspondants et/ou les produits induits par lesdits récepteurs qui peuvent donc avoir des activités pro ou anti.

Il convient de rappeler que les anticorps monoclonaux dirigés contre le récepteur Notch3 peuvent, suivant l'épitope reconnu, avoir une activité pro ou anti-Notch3, ce qui les rend utilisables dans des compositions thérapeutiques.

Enfin, la présente invention concerne, comme cela a été dit précédemment, plus particulièrement des méthodes de diagnostic d'une prédisposition à des affections neurologiques de type CADASIL, **caractérisées en ce qu**'on détermine à partir d'un prélèvement biologique dudit patient la présence d'une mutation qui supprime une cystéine ou qui fait apparaître une cystéine dans un des domaines EGF du polypeptide Notch3 dont la séquence est représentée à la figure 1, par l'analyse de tout ou partie d'une séquence nucléique correspondant audit gène, la présence d'au moins une telle mutation étant indicative d'une prédisposition dudit patient auxdites affections.

D'autres méthodes de diagnostic peuvent permettre de caractériser au moyen d'anticorps le dépôt prévu dans la membrane basale des cellules musculaires lisses vasculaires, dépôt qui pourrait être constitué de la protéine Notch3 elle-même ou d'un de ses produits de clivage.

Parmi les mutations qui sont recherchées, il faut citer plus particulièrement les mutations référencées dans le tableau C et la figure 3.

Les séquences d'acides nucléiques pourront être aussi bien de l'ADN génomique, un ADNc ou un ARNm.

Comme cela a été dit précédemment, parmi les troubles neurologiques qui peuvent être mis en évidence, on entend plus particulièrement les troubles de type cérébro-vasculaire et notamment le CADASIL, mais on a donné précédemment la liste de certains troubles qui pourraient être liés à une anomalie du récepteur Notch3 ; parmi ces affections il faut tout particulièrement citer l'implication potentielle de Notch3 dans les migraines avec ou sans aura et les démences d'étiologie actuellement inconnue.

Les outils diagnostiques basés sur la présente invention peuvent permettre un diagnostic positif et différentiel chez un malade pris isolément ou bien un diagnostic présymptomatique chez un sujet à risque (antécédent familial par exemple), il est possible également de prévoir un diagnostic anté-natal.

En outre, la mise en évidence d'une mutation spécifique peut permettre un diagnostic évolutif.

Les méthodes permettant de mettre en évidence la mutation dans un gène par rapport au gène naturel sont, bien entendu, très nombreuses, elles peuvent être mises en oeuvre par l'éude de l'ADN génomique, de l'ADNc et/ou de la protéine. On peut essentiellement les diviser en deux grandes catégories, le premier type de méthode est celui dans lequel la présence d'une mutation est détectée par comparaison de la séquence mutée avec la séquence correspondante naturelle non mutée, et le second type dans lequel la présence de la mutation est détectée de façon indirecte, par exemple par la mise en évidence de misappariements dûs à la présence de la mutation.

Dans les deux cas, on préférera en général les méthodes dans lesquelles tout ou partie de la séquence correspondant à Notch3 est amplifiée préalablement à la mise en évidence de la mutation, ces méthodes d'amplification peuvent être réalisées par des méthodes dites PCR (voir référence infra) ou PCR-like. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par la polymérase ; lorsque l'échantillons d'origine est un ARN il convient préalablement d'effectuer une transcription réverse, il existe actuellement de très nombreux procédés permettant cette amplification, par exemple les méthodes dites NASBA, TMA bien connues de l'homme du métier.

Le tableau B donne les séquences des amorces PCR qui permettent d'amplifier les exons ainsi que les températures des réactions PCR.

Une méthodologie générale d'amplification des séquences sera décrite dans les exemples.

### Recherche de mutations ponctuelles

Outre le séquençage direct de la mutation, différentes méthodes peuvent être utilisées. On citera brièvement les techniques :
1) recherche de "Single Strand Conformation Polymorphisms" (SSCP) (voir référence infra) ou Electrophorèse en gel en gradient dénaturant (DGGE).
2) les méthodes basées sur un clivage des régions misappariées (clivage enzymatique par la S1 nucléase, clivage chimique par différents composés tels que la pipéridine ou le tétroxide d'osmium, etc.
3) mise en évidence d'hétéroduplex en électrophorèse,
4) méthodes basées sur l'utilisation en hybridation de sondes oligonucléotidiques spécifiques d'allèles (ASO).

D'autres méthodes bien connues et basées sur les techniques d'hybrication peuvent être utilisées.

### Recherche de remaniements de type délétions, inversions, duplications

D'autres méthodes bien connues et basées sur les techniques d'hybridation à l'aide de sondes génomiques, de sondes ADNc, de sondes oligonucléotidiques, de ribosondes, de sondes dites de capture ou de sondes dites de détection, peuvent être utilisées pour la recherche de ce type de remaniements.

Une autre approche diagnostique utilisable lorsqu'on dispose de l'ADN de plusieurs sujets d'une même famille est basée sur la méthode d'analyse de liaison qui permet de calculer le risque qu'un sujet appartenant à une famille liée d'être porteur ou non du gène malade. Cette analyse peut être réalisée avec des marqueurs polymorphes situés à proximité immédiate du gène, ou des marqueurs polymorphes intragéniques.

Il est important de rappeler que, dans les familles CADASIL, l'existence de mutations dans le gène *Notch3* correspond à des mutations changeant des amino-acides essentiels pour la fonction de la protéine pour laquelle il code.

D'ailleurs, dans les exemples, on indique les situations des mutations actuellement détectées, mais il est possible que d'autres mutations existent dans le gène *Notch3* qui n'ont pas encore été mises en évidence mais qui doivent conduire aux mêmes types de risques sur le plan pathologique.

En tout état de cause, les protéines Notch3 mutées peuvent présenter une antigénicité différente de celle de la protéine naturelle.

Il est donc possible de réaliser un diagnostic ou pronostic d'une susceptibilité à des troubles neurologiques, en particulier cérébro- vasculaires de type CADASIL et des troubles liés au récepteur Notch3, en mettant en évidence le produit du gène muté de Notch3, ce type de détection est réalisable, par exemple, à l'aide d'anticorps monoclonaux ou polyclonaux. Dans ces conditions, il est possible de mettre en évidence et de doser le produit anormal du gène *Notch3* par des méthodes bien connues, RIA ou ELISA par exemple, ces technologies étant connues ne seront pas développées plus avant dans ce qui va suivre. Des anticorps dirigés contre la protéine normale pourrait aussi être utiles si le dépôt présent dans les artères de la peau correspondait à la protéine Notch3 ou à un de ses produits de clivage.

La présente invention concerne également une méthode de diagnostic in vitro de CADASIL mettant en oeuvre les anticorps monoclonaux ou polyclonaux marqués et correspondant à tout ou partie des protéines mutées sur des prélèvements biologiques.

Ainsi, il semble que les amas granulaires présents dans les basales des cellules musculaires lisses vasculaires sont dues à une accumulation de la protéine anormale et la recherche de cette protéine à l'aide d'anticorps, soit dans des biopsies soit in vivo, présente un intérêt diagnostique.

### Méthodes basées sur la détection du produit du gène

Les mutations du gène *Notch3* peuvent être responsables de différentes modifications du produit de ce gène, modifications utilisables pour une approche diagnostique. Brièvement, la protéine peut être tronquée, diminuée ou absente ; ses propriétés, en particulier d'antigénicité peuvent être modifiées. Toutes ces modifications peuvent être utilisées en approche diagnostique, grâce à plusieurs méthodes bien connues basées sur l'utilisation d'anticorps mono ou polyclonaux reconnaissant la protéine normale ou des variants mutés et ce à partir de l'étude d'extraits protéiques ou de coupes de tissus (par exemple biopsies de peau), ou d'études conduites in vivo (imagerie à l'aide d'anticorps couplées à une molécule détectable en imagerie de type PET-scan, etc.).

Les anticorps polyclonaux ou monoclonaux peuvent être obtenus par réaction immunologique d'un organisme humain ou animal avec un agent immunogène constitué par une protéine ou un polypeptide susceptible d'être obtenu à partir de cellules procaryotes ou eucaryotes transformées par un vecteur tel que décrit précédemment. Préférentiellement, l'agent immunogène est constitué par un polypeptide spécifique de la forme mutée de la protéine Notch dont la séquence est choisie parmi les séquences polypeptidiques comprenant au moins une mutation choisie parmi les mutations correspondant à la figure 3 ou au tableau C.

La présente invention décrit enfin, des compositions thérapeutiques comportant à titre de principe actif un composé à activité pro-Notch3, notamment tel que décrit précédemment, ainsi que des compositions thérapeutiques comportant à titre de principe actif un composé à activité anti-Notch3.

D'autres caractéristiques et avantages de la présente invention apparaîtront à la lecture des exemples ci-après, faite en se référant aux dessins annexés dans lesquels :
- la figure 1 reproduit la séquence d'ADNc de Notch3 humain ainsi que la séquence protéique correspondante,
- la figure 2 représente la structure générale du produit du gène *Notch3* ainsi que les mutations qui ont été mises en évidence par alignement des clones d'ADNc humains avec Notch3 de souris,
   A - gène *Notch3* de souris avec ses 34 domaines EGF, 3 répétitions Notch/Lin12 et 6 répétions cdc10, de même que le domaine transmembranaire,
   B - en bas, 8 des ADNc humains avec les identifications correspondant à la figure 1, en haut, l'alignement de certaines séquences génomiques avec l'ADNc d'au moins 29 exons,
      les origines des différents fragments apparaissent en bas de la figure 4 ;
      les différents clones sont disponibles commercialement ou par l'intermédiaire de banques :
      - les clones 261623 ; 153875 : 149693
         sont disponibles auprès du Consortium IMAGE ;
      - le clone C-32b03
         est disponible auprès de GENEXPRESS (Généthon, Evry, France) ;
      - les clones p28-20 ; CNA-20
         sont disponibles auprès de CLONTECH ;
- la figure 3 schématise la situation et la nature des mutations impliquées dans le CADASIL ;

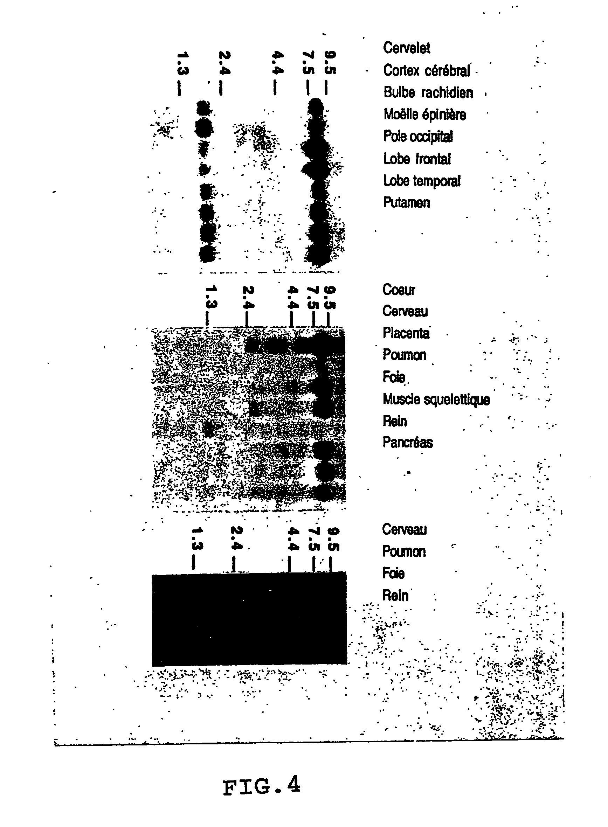

- la figure 4 représente une analyse "Northern blot",
   les Northern blots contenant 2 µg par ligne d'ADN poly (A⁺) humain, de gauche à droite :
   - de différents tissus de cerveaux,
   - de différents organes d'adultes,
   - de différents organes de foetus ;
ils sont hybridés avec p28-20, une sonde d'ADNc de 1,45 kb humain Notch3, on détecte un transcrit de 7,5 à 9,5 kb dans tous les tissus, aussi bien adultes que foetaux, à l'exception du foie, le transcrit est faiblement exprimé dans le tissu de cerveau au milieu et à droite ;
au contraire, à gauche on observe non seulement des transcrits de tous les tissus, mais également présence d'un transcrit compris entre 2,4 et 1,3 kb dont la présence n'a jamais été mentionnée et dont l'importance peut être très grande.

### EXEMPLE 1

### SCHEMA D'ISOLEMENT ET ANALYSE DU GENE Notch3

A la suite des remarques et de l'analyse qui ont été résumées au début de la description pour ce qui concerne la localisation du gène, on a utilisé des sondes de cDNA murin pour isoler le cDNA du gène *Notch3* humain, puis des clones génomiques dont les séquences pouvaient être alignées avec lui et avec les séquences du cDNA murin.

Des informations complémentaires sur les séquences ont été obtenues à partir d'un fragment de cDNA (884Na4) obtenu par cDNA sélection sur YAC (884g1) et de deux fragments génomiques (J431NH et J432NH) qui ont été obtenus par sous-clonage de fragments NotI-HindIII du BAC 13J4.

Dans le screening de la banque de données de (dBest) avec toutes les séquences, on a pu identifier des clones additionnels (clones IMAGE, Genexpress).

La séquence codante du gène humain *Notch*3, très fortement homologue avec le gène murin correspondant est représenté à la figure 1.

Le tableau A schématise la structure du gène en précisant la séquence et la position des jonctions exon-intron.

Dans ce tableau, le premier exon correspond à une séquence dont l'extrémité 5' n'a pas été entièrement clonée, de même pour l'exon 33.

Il faut remarquer qu'il peut exister des cDNA alternatifs correspondant au phénomène connu d'épissage alternatif.

Cette séquence contient 34 domaines EGF, 3 répétitions Notch/lin12, de même que 3 répétitions ankyrin like cdc10. Les protéines humaines et murines présentent 90,5% d'identité sur la séquence disponible actuellement. Une sonde cDNA
humaine partielle de 1,45 kb contenant les domaines EGF-like révèle un transcrit ubiquitaire dans les tissus foetaux, de même que dans les tissus humains adultes dont la taille comprise entre 7,5 et 9,5 kb est similaire au transcrit murin (figure 4).

Cette sonde révèle un autre transcrit dans certaines sous-régions du cerveau dont la taille est estimée être entre 1,3 et 2,4 kb (figure 4).

### EXEMPLE 2

### ETUDE DES MUTATIONS

Afin d'étudier l'importance des mutations dans le gène *Notch3* sur le CADASIL, on a tout d'abord étudié l'éventuelle présence de réarrangement d'ADN génomique important, en utilisant différentes combinaisons d'enzymes et des sondes Notch3.

Aucun réarrangement drastique n'a pu être mis en évidence chez les patients CADASIL, c'est pourquoi on a recherché ensuite des mutations ponctuelles.

Ainsi, on a étudié les mutations de la séquence codante totale du gène *Notch*3 dans l'ADN génomique en utilisant une combinaison de méthode SSCP et d'analyse hétéroduplex chez 51 patients CADASIL sans relation de parenté. 28 d'entre eux appartiennent à des familles pour lesquelles l'évidence d'un lien avec le chromosome 19 a été mis en évidence et 33 présentent des lésions ultrastructurales de la paroi des artérioles de la peau (présence de dépôts granulaires osmiophiles dans la membrane basale des cellules musculaires lisses vasculaires) .

Toutes les jonctions d'épissage, sauf 3, ont été analysées. En outre, un séquençage direct des produits de PCR de l'exon 4 et ses sites d'épissage a été effectué sur tous les patients.

On a trouvé des altérations qui sont compatibles avec des mutations correspondantes chez 42 patients (82%), lesdites mutations n'étant observées dans aucun des 200 chromosomes contrôles. Pour 26 des patients, il a été possible d'analyser un ou plusieurs apparentés, affectés ou non, et dans chaque cas on a établi que la mutation ségrégeait avec le phénotype CADASIL. Il y a 29 mutations différentes, dont 20 sont décrites pour la première fois. Elles incluent 24 mutations faux sens, qui apparaissent chez 40 patients, mutations qui doivent remplacer (16) un amino-acide par une cystéine additionnelle ou mutée (8) l'un des 6 résidus cystéine, qui sont les éléments clés des domaines EGF.

Deux mutations au site d'épissage 5' chez les deux derniers patients doivent normalement affecter l'épissage de l'exon 4. Les trois dernières mutations sont des mutations faux sens qui apparaissent chez 3 patients simultanément avec les mutations décrites précédemment.

Le patient 21 porte 2 mutations distinctes qui changent une arginine en cystéine au codon 141 dans l'EGF 3 et qui change un glycine conservée en alanine au codon 288 dans l'EGF 7. Le pedigree de ce patient n'était pas disponible, on n'a donc pas pu étudier la coségrégation de ces deux mutations.

Le patient 29 porte une première mutation dans l'EGF 4 qui change une arginine 182 en cystéine et une seconde qui change une alanine hautement conservée 1852 en thréonine dans le domaine cdc10. Ces deux mutations ségrègeat avec la maladie.

Le dernier patient 55 est porteur de deux mutations distinctes dans les domaines EGF, qui changent une cystéine (224) en un tryptophane et une leucine non conservée (497) en un résidu sérine.

Bien que ces trois dernières mutations faux sens ne soient pas détectées dans les 200 chromosomes contrôles, il peut s'agir de polymorphismes rares et compte tenu de la présence également des mutations faux sens qui mutent ou créent des résidus cystéines.

Il faut remarquer que la plus grande partie de ces 26 mutations ayant un effet pathogène résident exclusivement dans les parties EGF. 41% de ces mutations (11 sur 26) apparaissant chez 25 patients sont situées dans l'exon 4 et 65% (17 sur 26) résident dans les 6 premiers domaines EGF (voir en particulier la figure 3.

La figure 3 permet la mise en évidence de caractériser les principales mutations mises en évidence, la nomenclature retenue indique la position de la mutation ainsi que les modifications correspondantes de la protéine.

Comme cela a été indiqué précédemment, le fait qu'un gène Notch soit impliqué dans des désordres neurologiques de l'adulte paraît tout à fait étonnant puisque Notch est principalement connu pour intervenir au cours du développement chez la drosophile. Aucune des familles de CADASIL étudiées jusqu'à maintenant ne présente des anomalies du développement.

### EXEMPLE 3

### MISE EN EVIDENCE DES MUTATIONS CHEZ LES PATIENTS PAR LA METHODE SSCP

Les oligonucléotides utilisés comme amorces ont été synthétisés à partir des séquences jonctionnelles intron-exon (tableau B), de façon à amplifier des fragments génomiques d'environ 200 bp. Les séquences des amorces PCR sont données ci-après (tableau A).

Les analyses peuvent être réalisées à partir d'ADN extrait d'échantillons sanguins ou de tout autre tissu.

Les réactions d'amplification sont effectuées dans un volume final de 25 µl contenant 100 ng d'ADN génomique, 0,5 µm de chaque primer, 150 µg d'un mélange de 4dNTPs, la Taq polymérase 1XPCR de Cetus, 1 U de Taq polymérase (BRL), 1,5 µCi αdCTP marqué au P33 selon un protocole comportant 30 cycles identiques (94°C, 15 s ; 65°C, 15 s ; 72°C, 15 s).

Pour certaines des amorces, une température "d'annealing" de 70°C doit être utilisée, comme cela est indiqué dans le tableau A.

Les produits PCR sont dénaturés dans le formamide à 50 % et séparés par électrophorèse dans un gel de polyarylamide à 6 % non dénaturant.

Après autoradiographie, les bandes SSCP obtenues chez les patients sont comparées avec celles de contrôles sains à la recherche de variants anormaux. L'analyse de ceux-ci peut être utilisée comme approche diagnostique. Ces variants peuvent ensuite être séquencés si nécessaire.

**Tableau A**

| Structure exon-intron du gène Notch3 (séquences et positions des jonctions exon-intron) | | | |
|---|---|---|---|
| Site Intron / Exon accepteur d'épissage | Exon (Taille) | Position | Site Exon/ Intron donneur d'épissage |
| | 1 | 1-196 | CTGCAG/gtgaggggc laAlaAl |
| cccacacag/CCCCCC aProPr | 2 (79) | 197-275 | CTGCCT/gtgagtgcc acysLe |
| gcccacag/GTGCCC uCysPr | 3(143) | 276-418 | TCCGAG/gtgagagg heArgG |
| ccctccag/GCCCTG lyProA | 4 (339) | 419-757 | TTCCTG/gtgagtga euProG |
| cttgttag/GGTTTG lyPheG | 5 (123) | 758-880 | GGACAG/gtgggcac rpThrG |
| tgccacag/GCCAGT lyGlnP | 6 (234) | 881-1114 | AGACTG/gtgagtgg ysThrG |
| cttcccag/GCCTCC lyLeuL | 7 (156) | 1115-1270 | CTATCG/gtgagggg erIleG |
| tccggcag/GCGCCA lyAlaA | 8 (187) | 1271-1456 | TGGCAG/gtgggtgg etAlaG |
| tgcccccag/GCTTCA lyPheT | 9 (114) | 1457-1570 | CCTCGG/gtgaggac roSerG |
| caccccag/GCTTCA lyPheS | 10(114) | 1571-1684 | CCGAGG/gtgaggcg laGluG |
| ccccacag/GCTTTG lyPheG . | 11 (234) | 1685-1918 | CCACAG/gtgggacc hrThrG |
| gccccctag/GTGTGA lyValA | 12 (111) | 1919-2029 | TCACAG/gtgggcaa heThrG |
| ctcccccag/GGCCCC lyProL | 13 (193) | 2030-2222 | TGGCGG/gtgagggc oGlyGl |
| cctgccag/GTTCCG yPheAr | 14 (152) | 2223-2374 | TCCAGG/gtgtgtac alGlnG |
| cccaacag/GACGTC lyArgG | 15 (114) | 2375-2488 | GGCAAG/gtatgccac rpGlnG |
| tacccccag/GCCCAC lyProA | 16 (156) | 2489-2644 | ACCCCA/gtgagtgca spProA |
| gtccgcag/ACCCAT snProC | 17 (226) | 2645-2870 | CCCCAG/gtgggcgg rProSe |
| cgctccag/CTCCTG rSerCy | 18 (202) | 2871-3072 | TGCCAG/gtgggtgg CysGln |
| ccctccag/ACGCTG ThrLeu | 19 (148) | 3073-3220 | AGATCG/gtgagtgg lnIleG |
| ctttgcag/GGGTGC lyValA | 20 (185) | 3221-3405 | TGTGAG/gtaagggg CysGlu |
| cactgaag/TGTCTT CysLeu | 21 (133) | 3406-3538 | CGCTGG/gtatgcca hrLeuG |
| tccccccag/GGGTGC lyValL | 22 (258) | 3539-3796 | TCTCAG/gttaacct heSerG |
| tcgctcag/GTCCTC lyProA | 23 (119) | 3797-3915 | GCCCAG/gtaggtgtg AlaGln |
| gaccccccag/CCGTTC ProPhe | 24 (566) | 3916-4481 | TTGCAA/gtgagccc rCysAs |
| cccaccag/CCCGGT nProVa | 25 (333) | 4482-4814 | GATCGG/gtgagtgac lIleG |
| tccctgcag/CTCGGT ySerVal | 26 (155) | 4815-4969 | TGCGGG/gtgcggcc alArgG |
| tgctcttag/GGGAGC lyGluP | 27 (223) | 4970-5192 | CATGAA/gtgagaac yMetLy |
| tccgccag/GAACAT sAsnMe | 28 (85) | 5193-5277 | CTAAAG/gtactgcc LeuLys |
| cccctccag/GTAGAG ValGlu | 29 (162) | 5278-5440 | GCCCAG/gtcagtgac lyProA |
| ccctgcag/ATGGCT spGlyP | 30 (305) | 5441-5745 | TTCCAG/gtgagata PheGln |
| tgtcctag/ATTCTC IleLeu | 31 (148) | 5746-5893 | AGCTTG/gtaggttg luLeuG |
| ccctccag/GGAAAT lyLysS | 32 (99) | 5894-5992 | AGCAAG/gtgagccc SerLys |
| ccccccag/GAGGAG GluGlu | 33 | 5993- | |

**Tableau B**

| Séquences des primers utilisés pour le screening des mutations du gène Notch3 | | | | | |
|---|---|---|---|---|---|
| **Exon** | **Taille** | **Domaine** | | **Amorces** | **Taille du produit PCR** |
| 1 | | Peptide signal | EOF | AAGGAGGGAGGAGGGGAG | 125 |
| | | | EOR | TGGGGGTTCTTGCACTCC* | |
| | | | EOF | AAGGAGGGAGGAGGGGAG | 163 |
| | | | E0RBIS | GGTTCCTGCCTCCCATGA* | |
| | | | | | |
| 2 | 79 | EGF1 | E1F | TCCTCCACCTTCCTTCAC* | 148 |
| | | | E1R | ACACACAGGGCCCACTGGT* | |
| | | | | | |
| 3 | 143 | EGF 1-2 | N1F | TGTGCTGCCCAACCAAGCCA* | 224 |
| | | | N1R | ACTGACCACACCCCCGACTA* | |
| | | | | | |
| 4 | 339 | EGF 2-5 | N2A F | TAGTCGGGGGTGTGGTCAGT* | 192 |
| | | | N2A R | TCATCCACGTCGCTTCGGCA | |
| | | | | | |
| | | | CNA F | ATGGACGCTTCCTCTGCTC | 167 |
| | | | CNA R | ACATAGTGGCCCTGTGTAGC | |
| | | | | | |
| | | | CNA F | ATGGACGCTTCCTCTGCTCC | 295 |
| | | | N3AR | CCTCTGACTCTCCTGAGTAG* | |
| | | | | | |
| 5 | 123 | EGF 5-6 | N23Fbis | TGACCATCCTTGCCCCCTT* | 241 |
| | | | N23 R | CTGGCCTGTGGCACACAGAT * | |
| | | | | | |
| 6 | 234 | EGF6-8 | N13A F | TGGACTGCTGCATCTGTGTG* | 191 |
| | | | N13A R | ACACGCCTGTGGCACAGTCA | |
| | | | N13B F | GAGCTGCAGTCAGAATATCG | 145 |
| | | | N13B R | ATCCATGGCTCCCTGCAGAG* | |
| | | | | | |
| 7 | 156 | EGF 8-10 | N24 F | CAGAGCAGGAAGATCTGCCT* | 229 |
| | | | N24 R | CATTCACAGACGACGGAGCT* | |
| 8 | 187 | EGF 10-11 | N3 F | ATCGCACTCCATCCGGCA* | 212 |
| | | | N3R | ACCCACCTGCCATACAGA* | |
| | | | | | |
| 9 | 114 | EGF 11-12 | N25AF | CGTTCACACCATAGGGTAGC* | 215 |
| | | | N25A R | CCCCTTCCCAGACATGTCTT | |
| | | | | | |
| 10 | 114 | EGF 12-13 | N25BF | CTTGTCGGACTGTCATTGG | 195 |
| | | | N25BR | GTGTACTGCTCTCACCCTT* | |
| | | | | | |
| 11 | 234 | EGF 13-15 | N4AF | ATTGGTCCGAGGCCTCACTT* | 213 |
| | | | N4AR | ACCTGGCTCTCGCAGCGTGT | |
| | | | | | |
| | | | N4B R | CCATTCCCAACCCCTCTGTG | 199 |
| | | | N4B F | TGCCTGTGCTCCTGGCTACA* | |
| | | | | | |
| 12 | 111 | EGF 15-16 | N5 F | TGGCCACTCCATGCCATGTT* | 166 |
| | | | N5 R | TCTCATGGCAGCCACTTGCC* | |
| | | | | | |
| 13 | 193 | EGF 16-18 | N14 F | ATGAGTGTGCTTCCAGCCCA* | 258 |
| | | | N14R | GCAGTGTCTGAGGCTGAGAA* | |
| 14 | 152 | EGF 18-19 | N6 F | TCCCTGGCCTGACTACCTTC* | 207 |
| | | | N6 R | CTGCAGAGGGAAGGTGAGGT* | |
| | | | | | |
| 15 | 114 | EGF 19-20 | N26BF | AAGGCTATCCTGCTTCC* | 183 |
| | | | N26BR | GAGGAGGAGGGAAGAGAA* | |
| | | | | | |
| 16 | 156 | EGF 20 22 | S13FBIS | AGGATGTGGACGAGTGTGCT | 195 |
| | | | N26CR | GCTTAATGACTGTGTTCC* | |
| | | | | | |
| 17 | 226 | EGF 22-24 | N15A F | TCAGACTGGGCTAATGGGGG* | 257 |
| | | | N15A R | TCGCAGTGGAAGCCTCCGTA | |
| | | | | | |
| | | | N15B F | GATGTGGATGAGTGCCTGAG | 166 |
| | | | N15B R | GTCCTGCTCTTCAAGCAGA* | |
| | | | | | |
| 18 | 202 | EGF 24-25 | N27F | GATCCTCCCTCCCACTCCTT* | 256 |
| | | | N27R | AGGTCCCCAGTAACTCCA* | |
| | | | | | |
| 19 | 148 | EGF 25-27 | N22 F | ACTGACTCTAAGTGCTTCCC* | 240 |
| | | | N22 R | AGCAGGAGGTACGTGCATGA* | |
| | | | | | |
| 20 | 185 | EGF 27-28 | N7 F | TGTTCCTGTGCCACTCTCCT* | 249 |
| | | | N7 R | ACCTCCTCTTCCCTCTCCT* | |
| | | | | | |
| 21 | 133 | EGF 28-29 | N8 F | TCTGTGTCCCACTAAGCTGA* | 237 |
| | | | N8R | CAAGAGGAAATGAAGACAGC* | |
| | | | | | |
| 22 | 258 | EGF 29-31 | N9A F | TTCCTCTTGACCACCCCTCG* | 217 |
| | | | N9A R | TGGCAGGCACCTGAGCGACA | |
| | | | | | |
| | | | N9B F | CAGGATACACTGGTTTGCGC | 209 |
| | | | N9B R | TGCCACGTTATGGATCAGCC* | |
| | | | | | |
| 23 | 119 | EGF 31-32 | N10 F | GATCTACATGCTCCCGCTCG* | 178 |
| | | | N10R | TACTCCTCCTCCATAGGCCG* | |
| | | | | | |
| 24 | 566 | EGF 32-34 | N16AFTR | CGTTCTGGGGTCCGCGTT | 249 |
| | | Lin12 N1-3 | N16DR | AAGCGCAGCGGAAGAAGGGC | |
| | | | | | |
| | | | N16FF | GCCCTTCTTCCGCTGCGCTT | 230 |
| | | | N16FR | ACTGCAGCGCCTCGCATTGC | |
| | | | | | |
| | | | N16GF | CTGCGACCGCGAGTGCAACA | 239 |
| | | | N16HR | ATAGACAGACGGATCGAT* | |
| | | | | | |
| 25 | 331 | Lin12 N3 | N21CF | CTCTCTGCCTCACCCTT* | 207 |
| | | | N21CR | GCTGGAACGCAGTAGCT | |
| | | | N21DF | TGCTCACAGTGCTGCTG | 223 |
| | | | N21DR | CACGGCTTTTCCAGGTG* | |
| | | | | | |
| 26 | 155 | | N34F | TTTGAGCCCTCTGGTCC* | 306 |
| | | | N34R | AAGAGCAGGAAGCAGAG* | |
| 27 | 222 | TM | N28Fbis | TCCCTCTGCTTCCTGCTCTT* | 291 |
| | | | N28R | TCACAAGGTCCCCGTAGTCA* | |
| | | | | | |
| 28 | 85 | | JSN3 F | CTCACATCCCCTCTTCCCAT* | 203 |
| | | | J5N3R | ATCACGCCCATCATCCACTG* | |
| | | | | | |
| 29 | 163 | Cdc10 N1 | L24bisf | CAGCACCAAAGGGTG* | 241 |
| | | | L24bisR | CATCCCTTTGGGAGG* | |
| | | | | | |
| 30 | 305 | Cdc10 N1-3 | N17AF | ATGGCTTCACCCCGCTAATG | 176 |
| | | | N17AR | AGCCAGGTGCAAAGCAGTCT | |
| | | | | | |
| | | | N17BF | TCAGCTTGGGGCACGGACTG | 165 |
| | | | N17BR | GCATCGGCTGTGACAGCTGT | |
| | | | | | |
| 31 | 148 | Cdc10 N4-5 | N26FBIS | TGTTCCTGCCATGACCCCT* | 239 |
| | | | N26RBIS | CAGGTGACACTAACCCAGTC* | |
| | | | | | |
| 32 | 98 | Cdc10 N5-6 | N31F | TCCTGACCTCTCTCCCCTTC* | 178 |
| | | | N31R | AATGGCGCTGTGCCACTGCT* | |
| | | | | | |
| 33 | | Cdc10 N6 | N32AF | GCTACTGTTAGCTGGGGTTT* | 214 |
| | | NLS | N32AR | TGATCCAGCAAGCGCACGAT | |
| | | PEST | | | |
| | | | N32EFTER | TCACCGACCACCTGGACA | 425 |
| | | | N32DR | ACCAAGCTGTGCCAGAGA | |
| | | | | | |
| | | | N32DF | TCCAAGAAGAGCAGGAGG | 246 |
| | | | N32DR | ACCAAGCTGTGCCAGAGA | |
| | | | | | |
| | | | N32B F | CAGTGTCTCTGGCACAGCTT | 248 |
| | | | N32BR | TCCTGGGACTGCCAGGTAA | |
| | | | | | |
| | | | N32CF | AGCTGCTCAACCCAGGGA | 229 |
| | | | N32CR | GTGGATTCGGACCAGTCT | |
| | | | | | |
| | | | N32GF | GAATCCCCTGAGCACT | 235 |
| | | | N32GR | CTAAGAACTGACGAGC | |

| | | | | | |
|---|---|---|---|---|---|
| * Amorces introniques | | | | | |

**Tableau C**

| Mutations Notch3 chez des patients CADASIL | | | | | | | | |
|---|---|---|---|---|---|---|---|---|
| Patient | Evidence de liaison | Lésions CML | Notch3 nt^{a} | Mutation *Noich3* | Effet | Exon | Domaine | Ségrégation |
| 52* | nd | nd | 224 | TGT-->TAT | C₄₉-->Y* | N2 | EGF1 | nd |
| 56 | nd | + | 291 | TGG-->TGT | W₇₁-->C | N3 | EGF1 | nd |
| 11 | + | nd | 406 | CGT-->TGT | R₁₁₀-->C | N3 | EGF2 | + |
| 3 | + | + | 419(-2) | AG-->GG | épissage anormal of exon 4 ? | N4 | | + |
| 39 | nd | + | 419(-2) | AG-->CG | épissage anormal de l'exon 4 ? | N4 | | nd |
| 10 | + | + | 475 | CGC-->TGC | R₁₃₃-->C | N4 | EGF3 | + |
| 20 | nd | + | 475 | CGC-->TGC | R₁₃₃-->C | N4 | EGF3 | nd |
| 46 | + | nd | 475 | CGC-->TGC | R₁₃₃-->C | N4 | EGF3 | + |
| 6 | + | nd | 499 | CGC-->TGC | R₁₄₁-->C | N4 | EGF3 | + |
| 12 | + | + | 499 | CGC-->TGC | R₁₄₁-->C | N4 | EGF3 | + |
| 19 | + | nd | 499 | CGC-->TGC | R₁₄₁-->C | N4 | EGF3 | + |
| 21* | nd | + | 499 | CGC-->TGC | R₁₄₁-->C | N4 | EGF3 | nd |
| | | | *941* | *GGC-->GCG* | *G₂₈₈-->A** | *N5* | *EGF7* | *nd* |
| 38 | + | nd | 499 | CGC-->TGC | R₁₄₁-->C | N4 | EGF3 | + |
| 49 | + | + | 499 | CGC-->TGC | R₁₄₁-->C | N4 | EGF3 | + |
| 26 | + | + | 514 | TGC-->CGC | C₁₄₆-->R | N4 | EGF3 | + |
| 4 | + | + | 535 | CGC-->TGC | R₁₅₃-->C | N4 | EGF3 | + |
| 50 | nd | + | 535 | CGC-->TGC | R₁₅₃-->C | N4 | EGF3 | + |
| 9* | + | + | 583 | CGC-->TGC | R₁₆₉-->C* | N4 | EGF4 | + |
| 15* | + | nd | 583 | CGC-->TGC | R₁₆₉-->C* | N4 | EGF4 | + |
| 24 | + | nd | 583 | CGC-->TGC | R₁₆₉-->C | N4 | EGF4 | + |
| 36* | nd | + | 583 | CGC-->TGC | R₁₆₉-->C* | N4 | EGF4 | nd |
| 48 | nd | + | 583 | CGC-->TGC | R₁₆₉-->C | N4 | EGF4 | nd |
| 1 | + | nd | 589 | GGT-->TGT | G₁₇₁-->C | N4 | EGF4 | + |
| 45* | + | + | 622 | CGC-->TGC | R₁₈₂-->C* | N4 | EGF4 | + |
| 47* | nd | + | 622 | CGC-->TGC | R₁₈₂-->C* | N4 | EGF4 | nd |
| 29* | + | + | 622 | CGC-->TGC | R₁₈₂-->C | N4 | EGF4 | + |
| | | | *5632* | *GCT-->ACT* | *A₁₈₅₂-->T** | *N30* | *cdc10* | + |
| 41 | nd | + | 631 | TGT-->CGT | C₁₈₅-->R | N4 | EGF4 | nd |
| 57 | nd | + | 712 | TGC-->AGC | C₂₁₂-->S | N4 | EGF5 | nd |
| 8 | + | nd | 742 | TGT-->GGT | C₂₂₂-->G | N4 | EGF5 | + |
| 55 | nd | nd | 749 | TGT-->TAT | C₂₂₄-->Y | N4 | EGF5 | + |
| | | | *1568* | *TCG-->TTG* | *S₄₉₇-->L* | *N9* | *EGF12* | *-* |
| 14 | + | + | 851 | TAT-->TGT | Y₂₅₈-->C | N5 | EGF6 | + |
| 54* | nd | + | 1703 | TGT-->TAT | C₅₄₂-->Y* | N11 | EGF13 | nd |
| 17* | + | + | 1750 | CGC-->TGC | R₅₅₈-->C* | N11 | EGF14 | + |
| 18* | + | + | 1750 | CGC-->TGC | R₅₅₈-->C* | N11 | EGF14 | + |
| 31* | nd | + | 1810 | CGC-->TGC | R₅₇₈-->C* | N11 | EGF14 | + |
| 43 | nd | nd | 2260 | CGC-->TGC | R₇₂₈-->C | N14 | EGF18 | nd |
| 25 | + | + | 3031 | CGC-->TGC | R₉₈₅-->C | N18 | EGF25 | + |
| 42 | nd | + | 3031 | CGC-->TGC | R₉₈₅-->C | N18 | EGF25 | nd |
| 7 | + | nd | 3094 | CGC-->TGC | R₁₀₀₆-->C | N19 | EGF26 | + |
| 35 | nd | nd | 3169 | CGC-->TGC | R₁₀₃₁-->C | N19 | EGF26 | + |
| 33 | nd | nd | 3769 | CGC-->TGT | R₁₂₃₁-->C | N22 | EGF31 | nd |
| 58* | nd | + | 3859 | TGC-->CGC | C₁₂₆₁-->R* | N23 | EGF32 | nd |

| | | | | | | | | |
|---|---|---|---|---|---|---|---|---|
| * patient et mutation reportés précédemment⁷ CML : Cellule musculaire lisse | | | | | | | | |

### REFERENCES DES DIFFERENTS PROCEDES CITES PRECEDEMMENT

### Polymerase chain Reaction (PCR)

Saiki et al., Science, 239, p.487, 1988, + manuel de référence.

### SSCP

Orita et al., Proc. Natl. Acad. Sci. USA, 86, p.2766-2770, 1989.

### Techniques de détection de mutations basées sur la mise en évidence de misappariements

- clivage chimique
- clivage enzymatique (S1 nuclease)
- hétéroduplex
- Allele Specific Oligonucléotidique probes (ASO)

### références :

Cotton et al., Proc. Natl. Acad. Sci. USA, 85, 4397, 1988
Sherk et al., Proc. Natl. Acad. Sci. USA, 72, 989, 1975
Cariello, Hum. Genet., 42, 726, 1988

### Vecteurs de clonage et techniques de biologie moléculaire de base

- Current protocols in molecular biology, Eds F.M. Ausubel, R. Brent, R.E. Kingston, D.D. Moore, S.G. Seldman, J.A. Smith et K. Struhl, Published by Green Publishing Associates and Wiley Interscience, 1st edition 1987, John Wiley and sons
- Molecular cloning. A laboratory manual, J. Sambrook, EF Fritsch et T. Mariatis, 2nd edition 1989, Cold Spring Harbor Laboratory Press

## Revendications

1. Séquence nucléotidique isolée choisie parmi :
a) les séquences codant pour un polypeptide comprenant la séquence du polypeptide Notch3 humain représenté à la figure 1 ; et
b) la séquence nucléique de l'ADNc représenté à la figure 1 codant pour le polypeptide Notch3 humain,
**caractérisée en ce que** les séquences telles que définies en a) et b) comportent une mutation qui supprime une cystéine ou qui fait apparaître une cystéine sur ledit polypeptide Notch3 dans un de ses domaines EGF.

2. Séquence nucléotidique selon la revendication 1, **caractérisée en ce que** ladite mutation qui supprime une cystéine ou qui fait apparaître une cystéine sur le polypeptide Notch3 humain représenté à la figure 1 est choisie parmi le groupe de mutations C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C et C1261R.

3. Séquence nucléotidique selon la revendication 2, **caractérisée en ce que** ladite mutation qui supprime une cystéine ou qui fait apparaître une cystéine sur le polypeptide Notch3 humain représenté à la figure 1 est choisie parmi le groupe de mutations R133C, R141C, C146R, R153C, R169C, R182C, Y258C, R558C et R985C.

4. Vecteur de clonage ou d'expression dans une cellule hôte appropriée d'une séquence nucléotidique, **caractérisé en ce qu'**il comporte une séquence selon l'une des revendications 1 à 3.

5. Vecteur selon la revendication 4, **caractérisé en ce qu'**il comporte les éléments permettant l'expression desdites séquences dans ladite cellule hôte.

6. Vecteur selon l'une des revendications 4 ou 5, **caractérisé en ce qu'**il s'agit d'un vecteur à réplication autonome.

7. Vecteur selon l'une des revendications 4 ou 5, **caractérisé en ce qu'i**l s'agit d'un vecteur d'intégration chromosomique.

8. Vecteur selon l'une des revendications 4 ou 5, **caractérisé en ce qu'**il s'agit d'un vecteur viral.

9. Vecteur selon la revendication 8, **caractérisé en ce que** le vecteur est réalisé sur la base d'un adénovirus, d'un rétrovirus, d'un poxvirus ou d'un virus herpétique.

10. Cellule transformée par un vecteur selon l'une des revendications 4 à 9.

11. Cellule selon la revendication 10, **caractérisée en ce qu'**il s'agit d'une cellule procaryote.

12. Cellule selon la revendication 10, **caractérisée en ce qu'**il s'agit d'une cellule eucaryote.

13. Procédé de production d'une protéine codée par une séquence selon l'une des revendications 1 à 3, **caractérisé en ce qu'**on cultive une cellule selon l'une des revendications 10 à 12 et **en ce que** l'on récupère la protéine produite.

14. Protéine ou polypeptide codée par une séquence nucléique selon l'une des revendications 1 à 3.

15. Protéine ou polypeptide selon la revendication 14 de séquence telle que représentée à la figure 1 et comprenant une dite mutation qui supprime une cystéine ou qui fait apparaître une cystéine sur ledit polypeptide Notch3 dans un de ses domaines EGF.

16. Polypeptide selon la revendication 15, **caractérisé en ce que** ladite mutation est choisie parmi le groupe de mutations R133C, R141C, C146R, R153C, R169C, R182C, Y258C, R558C et R985C.

17. Méthode de diagnostic d'une prédisposition à des affections de type CADASIL chez un patient, **caractérisée en ce qu'**on détermine à partir d'un prélèvement biologique dudit patient la présence d'une mutation qui supprime une cystéine ou qui fait apparaître une cystéine dans un des domaines EGF du polypeptide Notch3 dont la séquence est représentée à la figure 1, par l'analyse de tout ou partie de la séquence nucléique dont la séquence est représentée à la figure 1, la présence d'au moins une telle mutation étant indicative d'une prédisposition dudit patient auxdites affections.

18. Méthode de diagnostic selon la revendication 17, **caractérisée en ce que** la mutation que l'on cherche à déterminer est une mutation choisie parmi le groupe de mutations C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C et C1261R.

19. Méthode selon l'une des revendications 17 ou 18, dans laquelle la séquence d'acide nucléique analysée est un ADN génomique, un ADNc ou un ARNm.

20. Méthode selon l'une des revendications 17 à 19, **caractérisée en ce que** ladite analyse est réalisée par hybridation.

21. Méthode selon l'une des revendications 17 à 20, **caractérisée en ce que** la présence d'une mutation est détectée par comparaison avec la séquence correspondante naturelle non mutée.

22. Méthode selon la revendication 20, **caractérisée en ce que** ladite hybridation est réalisée à l'aide d'au moins une sonde oligonucléotidique spécifique du variant allélique.

23. Méthode selon l'une des revendications 17 à 21, **caractérisée en ce que** ladite analyse est réalisée par séquençage.

24. Méthode selon l'une des revendications 17 à 21, **caractérisée en ce que** ladite analyse est réalisée par migration électrophorétique.

25. Méthode selon la revendication 24, **caractérisée en ce que** ladite analyse réalisée par migration électrophorétique est réalisée par SSCP ou DGGE.

26. Méthode selon l'une des revendications 17 à 25, **caractérisée en ce que** tout ou partie de la séquence nucléique du gène Notch3 de séquence nucléique correspondant à la figure 1 est amplifiée préalablement à la mise en évidence de ladite mutation.

27. Méthode selon la revendication 26, **caractérisée en ce que** l'amplification est réalisée par PCR ou PCR-like.

28. Méthode selon la revendication 26 ou 27, **caractérisée en ce que** les amorces choisies pour réaliser l'amplification sont choisies parmi :
- les séquences jonctions entre les introns et les exons du gène Notch3 telles que représentées dans le tableau A ;
- les fragments de séquences telles que définies en a) et b) de la revendication 1, ces fragments ayant au moins 10, 20 ou 30 bases et comportant ladite mutation qui supprime une cystéine ou qui fait apparaître une cystéine dans un des domaines EGF dudit polypeptide Notch3 ; et
- les séquences telles que représentées dans le tableau B et leur séquence complémentaire.

29. Méthode de diagnostic *in vitro* de CADASIL **caractérisée en ce qu'**elle met en oeuvre une séquence nucléotidique comme amorce spécifique ou sonde spécifique d'un variant allélique du gène Notch3 humain ayant la séquence nucléotidique de la figure 1 cette séquence nucléotidique étant choisie parmi les séquences suivantes :
- les séquences jonctions entre les introns et les exons du gène Notch3 telles que représentées dans le tableau A ;
- les fragments de séquences telles que définies en a) et b) de la revendication 1, ces fragments ayant au moins 10, 20 ou 30 bases et comportant ladite mutation qui supprime une cystéine ou qui fait apparaître une cystéine dans un des domaines EGF dudit polypeptide Notch3 ; et
- les séquences telles que représentées dans le tableau B et leur séquence complémentaire.

30. Séquence nucléotidique utilisable comme amorce spécifique ou sonde spécifique d'un variant allélique du gène Notch3 humain ayant la séquence nucléotidique de la figure 1 dans une méthode diagnostique de CADASIL, **caractérisée en ce qu'**elle est choisie parmi les séquences suivantes :
- les fragments de séquences telles que définies en a) et b) de la revendication 1, ces fragments ayant au moins 20 bases et comportant ladite mutation qui supprime une cystéine ou qui fait apparaître une cystéine dans un des domaines EGF dudit polypeptide Notch3.

31. Méthode de diagnostic d'une prédisposition à des affections de type CADASIL chez un patient, **caractérisée en ce qu'**on détermine à partir d'un prélèvement biologique dudit patient la présence d'une mutation qui supprime une cystéine ou qui fait apparaître une cystéine dans un des domaines EGF du polypeptide Notch3 dont la séquence est représentée à la figure 1, la présence d'au moins une telle mutation étant indicative d'une prédisposition dudit patient auxdites affections.

32. Méthode de diagnostic selon la revendication 31, **caractérisée en ce que** la mutation que l'on cherche à déterminer est une mutation choisie parmi le groupe de mutations C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C et C1261R.

33. Méthode selon l'une des revendications 31 ou 32, **caractérisée en ce que** la détection est effectuée par un procédé ELISA ou RIA.

34. Méthode de diagnostic *in vitro* de CADASIL **caractérisée en ce qu'**elle met en oeuvre un anticorps polyclonal ou monoclonal reconnaissant la protéine Notch 3 humaine normale ou des variants mutés selon l'une des revendications 14 à 16.

## Claims

1. Isolated nucleotide sequence chosen from:
a) the sequences encoding a polypeptide comprising the sequence of the human Notch3 polypeptide represented in Figure 1; and
b) the nucleic sequence of the cDNA represented in Figure 1 encoding the human Notch3 polypeptide,
**characterized in that** the sequences as defined in a) and b) comprise a mutation which removes a cysteine from or which causes a cysteine to appear on the said Notch3 polypeptide in one of its EGF domains.

2. Nucleotide sequence according to Claim 1, **characterized in that** the said mutation which removes a cysteine from or which causes a cysteine to appear on the human Notch3 polypeptide represented in Figure 1 is chosen from the group of mutations C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C and C1261R.

3. Nucleotide sequence according to Claim 2, **characterized in that** the said mutation which removes a cysteine from or which causes a cysteine to appear on the human Notch3 polypeptide represented in Figure 1 is chosen from the group of mutations R133C, R141C, C146R, R153C, R169C, R182C, Y258C, R558C and R985C.

4. Vector for the cloning or expression, in an appropriate host cell, of a nucleotide sequence, **characterized in that** it comprises a sequence according to one of Claims 1 to 3.

5. Vector according to Claim 4, **characterized in that** it comprises the elements allowing the expression of said sequences in said host cell.

6. Vector according to either of Claims 4 and 5, **characterized in that** it is an autonomously replicating vector.

7. Vector according to either of Claims 4 and 5, **characterized in that** it is a chromosomal integrating vector.

8. Vector according to either of Claims 4 and 5, **characterized in that** it is a viral vector.

9. Vector according to Claim 8, **characterized in that** the vector is prepared based on an adenovirus, a retrovirus, a poxvirus or a herpesvirus.

10. Cell transformed with a vector according to one of Claims 4 to 9.

11. Cell according to Claim 10, **characterized in that** it is a prokaryotic cell.

12. Cell according to Claim 10, **characterized in that** it is a eukaryotic cell.

13. Method of producing a protein encoded by a sequence according to one of Claims 1 to 3, **characterized in that** a cell according to one of Claims 10 to 12 is cultured and **in that** the protein produced is recovered.

14. Protein or polypeptide encoded by a nucleic sequence according to one of Claims 1 to 3.

15. Protein or polypeptide according to Claim 14 having a sequence as represented in Figure 1 and comprising a said mutation which removes a cysteine from or which causes a cysteine to appear on the said Notch3 polypeptide in one of its EGF domains.

16. Polypeptide according to Claim 15, **characterized in that** the said mutation is chosen from the group of mutations R133C, R141C, C146R, R153C, R169C, R182C, Y258C, R558C and R985C.

17. Method of diagnosing a predisposition to conditions of the CADASIL type in a patient, **characterized in that** the presence of a mutation which removes a cysteine from or which causes a cysteine to appear in one of the EGF domains of the Notch3 polypeptide whose sequence is represented in Figure 1 is determined using a biological sample from said patient by analysis of all or part of the nucleic sequence whose sequence is represented in Figure 1, the presence of at least one such mutation being indicative of a predisposition of said patient to said conditions.

18. Diagnostic method according to Claim 17, **characterized in that** the mutation) which it is sought to determine is a mutation chosen from the group of mutations C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C and C1261R.

19. Method according to either of Claims 17 and 18, in which the nucleic acid sequence analyzed is a genomic DNA, a cDNA or an mRNA.

20. Method according to one of Claims 17 to 19, **characterized in that** said analysis is carried out by hybridization.

21. Method according to one of Claims 17 to 20, **characterized in that** the presence of a mutation is detected by comparison with the corresponding nonmutated natural sequence.

22. Method according to Claim 20, **characterized in that** said hybridization is carried out with the aid of at least one oligonucleotide probe specific for the allelic variant.

23. Method according to one of Claims 17 to 21, **characterized in that** said analysis is carried out by sequencing.

24. Method according to one of Claims 17 to 21, **characterized in that** said analysis is carried out by electrophoretic migration.

25. Method according to Claim 24, **characterized in that** said analysis carried out by electrophoretic migration is carried out by SSCP or DGGE.

26. Method according to one of Claims 17 to 25, **characterized in that** all or part of the nucleic sequence of the Notch3 gene having a nucleic sequence corresponding to Figure 1 is amplified prior to the detection of the said mutation.

27. Method according to Claim 26, **characterized in that** the amplification is carried out by PCR or PCR-like.

28. Method according to Claim 26 or 27, **characterized in that** the primers chosen to carry out the amplification are chosen:
- the joining sequences between the introns and the exons of the Notch3 gene as represented in Table A;
- the fragments of sequences as defined in a) and b) of Claim 1, these fragments having at least 10, 20 or 30 bases and comprising said mutation which removes a cysteine from or which causes a cysteine to appear in one of the EGF domains of said Notch3 polypeptide; and
- the sequences as represented in Table B and their complementary sequence.

29. Method of diagnosing CADASIL *in vitro,* **characterized in that** it uses a nucleotide sequence as specific primer or specific probe for an allelic variant of the human Notch3 gene having the nucleotide sequence of Figure 1, this nucleotide sequence being chosen from the following sequences:
- the joining sequences between the introns and the exons of the Notch3 gene as represented in Table A;
- the fragments of sequences as defined in a) and b) of Claim 1, these fragments having at least 10, 20 or 30 bases and comprising said mutation which removes a cysteine from or which causes a cysteine to appear in one of the EGF domains of said Notch3 polypeptide; and
- the sequences as represented in Table B and their complementary sequence.

30. Nucleotide sequence which can be used as a specific primer or a specific probe for an allelic variant of the human Notch3 gene having the nucleotide sequence of Figure 1 in a diagnostic method for CADASIL, **characterized in that** it is chosen from the following sequences:
- the fragments of sequences as defined in a) and b) of Claim 1, these fragments having at least 20 bases and comprising said mutation which removes a cysteine from or which causes a cysteine to appear in one of the EGF domains of said Notch3 polypeptide.

31. Method of diagnosing a predisposition to CADASIL-type conditions in a patient, **characterized in that** the presence of a mutation which removes a cysteine or which causes a cysteine to appear in one of the EGF domains of the Notch3 polypeptide whose sequence is represented in Figure 1 is determined using a biological sample from said patient, the presence of at least one such mutation being indicative of a predisposition of said patient to said conditions.

32. Diagnostic method according to Claim 31, **characterized in that** the mutation which it is sought to determine is a mutation chosen from the group of mutations C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C and C1261R.

33. Method according to either of Claims 31 and 32, **characterized in that** the detection is carried out by an ELISA or RIA method.

34. Method of diagnosing CADASIL in vitro, **characterized in that** it uses a polyclonal or monoclonal antibody recognizing the normal human Notch3 protein or mutated variants according to one of Claims 14 to 16.

## Patentansprüche

1. Isolierte Nukleotidsequenz, ausgewählt aus:
a) den Sequenzen, die für ein Polypeptid kodieren, die die Sequenz des humanen Polypeptids Notch3 enthalten, das auf der Figur 1 dargestellt ist, und
b) der cDNA-Nukleotidsequenz, die auf der Figur 1 dargestellt ist, die für das humane Polypeptid Notch3 kodiert,
**dadurch gekennzeichnet, dass** die wie in a) und b) definierten Sequenzen eine Mutation aufweisen, die ein Cystein supprimiert oder die ein Cystein auf dem Polypeptid Notch3 in einer seiner EGF-Domänen erscheinen lässt.

2. Nukleotidsequenz nach Anspruch 1, **dadurch gekennzeichnet, dass** die Mutation, die ein Cystein supprimiert oder die ein Cystein auf dem humanen Polypeptid Notch3, das auf der Figur 1 dargestellt ist, erscheinen lässt, aus der Gruppe der Mutationen C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C und C1261R ausgewählt ist.

3. Nukleotidsequenz nach Anspruch 2, **dadurch gekennzeichnet, dass** die Mutation, die ein Cystein supprimiert oder die ein Cystein auf dem humanen Polypeptid Notch3, das auf der Figur 1 dargestellt ist, erscheinen lässt, aus der Gruppe der Mutationen R133C, R141C, C146R, R153C, R169C, R182C, Y258C, R558C und R985C ausgewählt ist.

4. Klonierungs- oder Expressionsvektor in einer geeigneten Wirtszelle einer Nukleotidsequenz, **dadurch gekennzeichnet, dass** er eine Sequenz nach einem der Ansprüche 1 bis 3 aufweist.

5. Vektor nach Anspruch 4, **dadurch gekennzeichnet, dass** er die Elemente aufweist, die die Expression der Sequenzen in der Wirtszelle erlauben.

6. Vektor nach einem der Ansprüche 4 oder 5, **dadurch gekennzeichnet, dass** es sich um einen Vektor mit autonomer Replikation handelt.

7. Vektor nach einem der Ansprüche 4 oder 5, **dadurch gekennzeichnet, dass** es sich um einen Chromosomen-Integrationsvektor handelt.

8. Vektor nach einem der Ansprüche 4 oder 5, **dadurch gekennzeichnet, dass** es sich um einen viralen Vektor handelt.

9. Vektor nach Anspruch 8, **dadurch gekennzeichnet, dass** der Vektor auf Basis eines Adenovirus, eines Retrovirus, eines Poxvirus oder eines Herpesvirus realisiert ist.

10. Zelle, die von einem Vektor nach einem der Ansprüche 4 bis 9 transformiert ist.

11. Zelle nach Anspruch 10, **dadurch gekennzeichnet, dass** es sich um eine prokaryotische Zelle handelt.

12. Zelle nach Anspruch 10, **dadurch gekennzeichnet, dass** es sich um eine eukaryotische Zelle handelt.

13. Verfahren zur Herstellung eines von einer Sequenz nach einem der Ansprüche 1 bis 3 kodierten Proteins, **dadurch gekennzeichnet, dass** eine Zelle nach einem der Ansprüche 10 bis 12 kultiviert wird und **dadurch**, dass das hergestellte Protein gewonnen wird.

14. Protein oder Polypeptid, das von einer Nukleinsequenz nach einem der Ansprüche 1 bis 3 kodiert ist.

15. Protein oder Polypeptid nach Anspruch 14 einer Sequenz wie auf der Figur 1 dargestellt und eine Mutation umfassend, die ein Cystein supprimiert oder die ein Cystein auf dem Polypeptid Notch3 in einer seiner EGF-Domänen erscheinen lässt.

16. Polypeptid nach Anspruch 15, **dadurch gekennzeichnet, dass** die Mutation aus der Gruppe der Mutationen R133C, R141C, C146R, R153C, R169C, R182C, Y258C, R558C und R985C ausgewählt ist.

17. Verfahren zur Diagnose einer Veranlagung zu Erkrankungen vom Typ CADASIL bei einem Patienten, **dadurch gekennzeichnet, dass** ausgehend von einer biologischen Probe des Patienten das Vorhandensein einer Mutation festgestellt wird, die ein Cystein supprimiert oder die ein Cystein in einer der EGF-Domänen des Polypeptids Notch3 erscheinen lässt, dessen Sequenz auf der Figur 1 dargestellt ist, durch Analyse der gesamten oder eines Teils der Nukleinsequenz, deren Sequenz auf der Figur 1 dargestellt ist, wobei das Vorhandensein von mindestens einer derartigen Mutation auf eine Veranlagung des Patienten zu diesen Erkrankungen hinweist.

18. Diagnoseverfahren nach Anspruch 17, **dadurch gekennzeichnet, dass** die Mutation, die man bestimmen möchte, eine Mutation ist, die aus der Gruppe der Mutationen C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C und C1261R ausgewählt ist.

19. Verfahren nach einem der Ansprüche 17 oder 18, wobei die analysierte Nukleinsäuresequenz eine genomische DNA, eine cDNA oder eine mRNA ist.

20. Verfahren nach einem der Ansprüche 17 bis 19, **dadurch gekennzeichnet, dass** die Analyse durch Hybridisierung durchgeführt wird.

21. Verfahren nach einem der Ansprüche 17 bis 20, **dadurch gekennzeichnet, dass** das Vorhandensein einer Mutation durch Vergleich mit der entsprechenden natürlichen nicht mutierten Sequenz nachgewiesen wird.

22. Verfahren nach Anspruch 20, **dadurch gekennzeichnet, dass** die Hybridisierung mit Hilfe von mindestens einer spezifischen Oligonukleotidsonde der Allelvariante durchgeführt wird.

23. Verfahren nach einem der Ansprüche 17 bis 21, **dadurch gekennzeichnet, dass** die Analyse durch Sequenzierung durchgeführt wird.

24. Verfahren nach einem der Ansprüche 17 bis 21, **dadurch gekennzeichnet, dass** die Analyse durch elektrophoretische Migration durchgeführt wird.

25. Verfahren nach Anspruch 24, **dadurch gekennzeichnet, dass** die durch elektrophoretische Migration durchgeführte Analyse durch SSCP oder DGGE durchgeführt wird.

26. Verfahren nach einem der Ansprüche 17 bis 25, **dadurch gekennzeichnet, dass** die gesamte oder ein Teil der Nukleinsequenz des Gens Notch3 der Nukleinsequenz, die der Figur 1 entspricht, vor dem Nachweis der Mutation amplifiziert wird.

27. Verfahren nach Anspruch 26, **dadurch gekennzeichnet, dass** die Amplifizierung durch PCR oder PCR-like durchgeführt wird.

28. Verfahren nach Anspruch 26 oder 27, **dadurch gekennzeichnet, dass** die zur Durchführung der Amplifizierung ausgewählten Primer ausgewählt sind aus:
- den Sequenzverbindungen zwischen den Intronen und den Extronen des Gens Notch3, wie in Tabelle A dargestellt,
- den Sequenzfragmenten, wie in a) und b) von Anspruch 1 definiert, wobei diese Fragmente mindestens 10, 20 oder 30 Basen haben und die Mutation, die ein Cystein supprimiert oder ein Cystein in einer der EGF-Domänen des Polypeptids Notch3 erscheinen lässt, aufweist, und
- den Sequenzen, wie in der Tabelle B dargestellt und ihrer komplementären Sequenz.

29. Verfahren zur in vitro-Diagnose von CADASIL, **dadurch gekennzeichnet, dass** es eine Nukleinsequenz als spezifischen Primer oder spezifische Sonde einer Allelvariante des humanen Gens Notch3 umsetzt, das die Nukleotidsequenz der Figur 1 hat, wobei diese Nukleotidsequenz ausgewählt ist aus den folgenden Sequenzen:
- den Sequenzverbindungen zwischen den Intronen und den Extronen des Gens Notch3, sowie in Tabelle A dargestellt,
- den Sequenzfragmenten, wie in a) und b) von Anspruch 1 definiert, wobei diese Fragmente mindestens 10, 20 oder 30 Basen haben und die Mutation, die ein Cystein supprimiert oder ein Cystein in einer der EGF-Domänen des Polypeptids Notch3 erscheinen lässt, aufweist, und
- den Sequenzen, wie in der Tabelle B dargestellt und ihrer komplementären Sequenz.

30. Nukleotidsequenz, die als spezifischer Primer oder spezifische Sonde einer Allelvariante des humanen Gens Notch3, das die Nukleotidsequenz der Figur 1 hat, in einem CADASIL-Diagnoseverfahren verwendbar ist, **dadurch gekennzeichnet, dass** sie ausgewählt ist aus den folgenden Sequenzen:
- den Sequenzfragmenten, wie in a) und b) von Anspruch 1 definiert, wobei diese Fragmente mindestens 20 Basen haben und die Mutation, die ein Cystein supprimiert oder ein Cystein in einer der EGF-Domänen des Polypeptids Notch3 erscheinen lässt, aufweist.

31. Verfahren zur Diagnose einer Veranlagung zu Erkrankungen vom Typ CADASIL bei einem Patienten, **dadurch gekennzeichnet, dass** ausgehend von einer biologischen Probe des Patienten das Vorhandensein einer Mutation festgestellt wird, die ein Cystein supprimiert oder die ein Cystein in einer der EGF-Domänen des Polypeptids Notch3 erscheinen lässt, dessen Sequenz auf der Figur 1 dargestellt ist, wobei das Vorhandensein von mindestens einer derartigen Mutation auf eine Veranlagung des Patienten zu diesen Erkrankungen hinweist.

32. Diagnoseverfahren nach Anspruch 31, **dadurch gekennzeichnet, dass** die Mutation, die zu bestimmen versucht wird, eine Mutation ist, die aus der Gruppe der Mutationen C49Y, W71C, R110C, R133C, R141C, C146R, R153C, R169C, G171C, R182C, C185R, C212S, C222G, C224Y, Y258C, C542Y, R558C, R578C, R728C, R985C, R1006C, R1031C, R1231C und C1261R ausgewählt ist.

33. Verfahren nach einem der Ansprüche 31 oder 32, **dadurch gekennzeichnet, dass** der Nachweis durch ein ELISA- oder RIA-Verfahren durchgeführt wird.

34. Verfahren zur in vitro-Diagnose von CADASIL, **dadurch gekennzeichnet, dass** es einen polyklonalen oder monoklonalen Antikörper umsetzt, der das normale humane Protein Notch3 oder nach einem der Ansprüche 14 bis 16 mutierte Varianten erkennt.
